(19) Europäisches Patentamt
European Patent Office
Office européen des brevets

(11) EP 1 574 206 A2

(12) DEMANDE DE BREVET EUROPEEN

(43) Date de publication:
**14.09.2005 Bulletin 2005/37**

(51) Int Cl.[7]: **A61K 7/13**

(21) Numéro de dépôt: **05290442.2**

(22) Date de dépôt: **25.02.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **27.02.2004 FR 0450382**
**25.06.2004 FR 0407020**

(71) Demandeur: **L'OREAL**
**75008 Paris (FR)**

(72) Inventeurs:
- **Greaves, Andrew**
  **77144 Montevrain (FR)**
- **Samian, Henri**
  **91570 Bievres (FR)**

(74) Mandataire: **Wattremez, Catherine**
**L'OREAL - D.I.P.I.**
**25-29 Quai Aulagnier**
**92600 Asnières (FR)**

# (54) Composition comprenant un colorant mixte à base d'au moins un chromophore de type nitre (hétéro)aromatique ou azine cyclique

(57) La présente invention a pour objet une composition tinctoriale comprenant au moins un colorant mixte comprenant au moins un chromophore de type azine et/ou de type nitré (hétéro)aromatique éventuellement associé à au moins un chromophore de type méthine et/ou carbonyle.

Elle concerne de plus un procédé de teinture de fibres kératiniques, notamment humaines, telles que les cheveux, mettant en oeuvre ladite composition, ainsi qu'un dispositif approprié.

Elle concerne enfin les colorants mixtes précités.

EP 1 574 206 A2

## Description

La présente invention a pour objet une composition tinctoriale comprenant un ou plusieurs colorants mixtes comprenant au moins un chromophore de type azine cyclique et/ou de type nitré (hétéro)aromatique, ainsi qu'un procédé de coloration de fibres kératiniques, notamment humaines, mettant en jeu ladite composition. Elle a de même pour objet des colorants mixtes en tant que tels.

Il est connu de teindre les fibres kératiniques et en particulier les cheveux humains avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique azinique ou triarylméthane.

Habituellement, ces colorants sont appliqués sur les fibres, éventuellement en présence d'un agent oxydant, si l'on souhaite obtenir un effet simultané d'éclaircissement des fibres. Une fois le temps de pose écoulé, les fibres sont rincées, éventuellement lavées et séchées.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur relative mauvaise tenue aux lavages ou à la transpiration.

On est aussi confronté à une difficulté supplémentaire, liée au fait que pour obtenir une couleur particulière, il est nécessaire dans la majeure partie des cas, pour ne pas dire la totalité, de mélanger plusieurs colorants. Or chaque colorant ne possède pas la même affinité pour la fibre, ce qui se traduit soit par des colorations hétérogènes, soit par des virages de couleur au cours du temps, par exemple après un ou plusieurs lavages des fibres, exposition au soleil, etc.

Le but de la présente invention est de fournir des colorants directs ne présentant pas les inconvénients des colorants directs existants.

En particulier, l'un des buts de la présente invention est de fournir des colorants directs qui permettent d'obtenir des nuances variées sans problème de virage de la couleur dans le temps.

Ces buts et d'autres sont atteints par la présente invention qui a pour objet une composition tinctoriale comprenant dans un milieu approprié pour la teinture des fibres kératiniques, notamment humaines, au moins un colorant mixte comprenant au moins deux chromophores différents ; au moins l'un des chromophores étant choisi parmi les chromophores de la famille des composés azines cycliques et/ou nitrés (hétéro)aromatiques, éventuellement associé à au moins un chromophore de la famille des méthines et/ou des carbonyles ; les chromophores étant liés entre eux par l'intermédiaire d'au moins un bras de liaison stoppant la délocalisation des électrons de chacun des chromophores ; à l'exception d'une composition comprenant un colorant mixte à deux chromophores dont l'un est un composé nitré benzénique, l'autre un composé nitré benzénique ou anthraquinonique ; les deux chromophores étant reliés au moyen d'un atome d'azote par l'intermédiaire d'un bras de liaison constitué par un radical alkyle éventuellement interrompu par un atome d'azote porteur d'un ou de deux radicaux choisis parmi l'hydrogène, un radical alkyle en $C_1$-$C_4$, un radical hydroxyalkyle en $C_1$-$C_4$. De préférence, ne sont pas couverts par l'invention les colorants mixtes à deux chromophores dont l'un est un composé nitré benzénique, l'autre un composé nitré benzénique ou anthraquinonique ; les deux chromophores étant reliés par l'intermédiaire d'un bras de liaison constitué par un radical alkyle éventuellement interrompu par un atome d'azote porteur d'un ou de deux radicaux choisis parmi l'hydrogène, un radical alkyle en $C_1$-$C_4$, un radical hydroxyalkyle en $C_1$-$C_4$. Ne sont de même pas compris dans l'invention les composés de formules suivantes

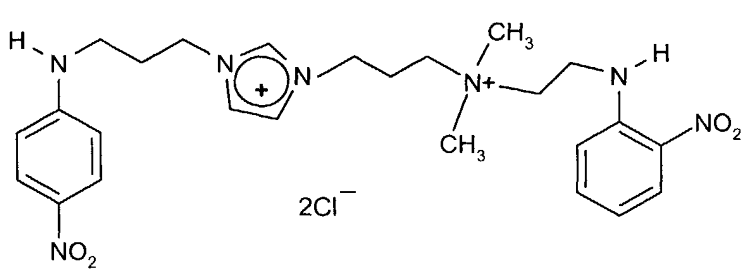

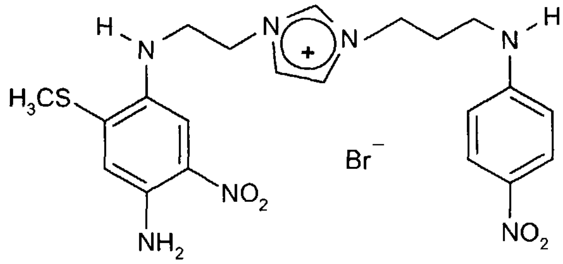

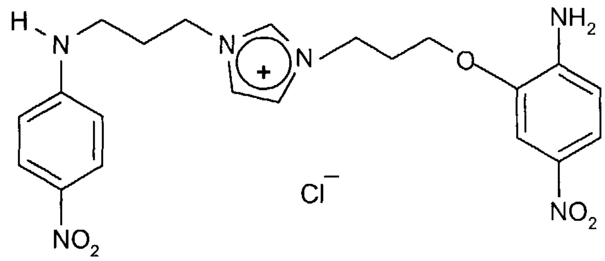

Elle a de plus pour objet un procédé de coloration de fibres kératiniques, notamment humaines, dans lequel on applique la composition tinctoriale précitée, éventuellement en présence d'au moins un agent oxydant, on laisse poser pendant une durée suffisante pour obtenir la coloration souhaitée, on rince éventuellement les fibres, on les lave éventuellement et on les rince, puis on les sèche ou on les laisse sécher.

Elle a enfin pour objet un colorant mixte comprenant au moins deux chromophores différents ; au moins l'un des chromophores étant choisi parmi les chromophores de la famille des composés azines cycliques et/ou nitrés (hétéro)aromatiques, éventuellement associé à au moins un chromophore de la famille des méthines et/ou des carbonyles ; les chromophores étant liés entre eux par l'intermédiaire d'au moins un bras de liaison stoppant la délocalisation des électrons de chacun des chromophores ; à l'exception d'un colorant mixte à deux chromophores dont l'un est un composé nitré benzénique, l'autre un composé nitré benzénique ou anthraquinonique ; les deux chromophores étant reliés au moyen d'un atome d'azote par l'intermédiaire d'un bras de liaison constitué par un radical alkyle éventuellement interrompu par un atome d'azote porteur d'un ou de deux radicaux choisis parmi l'hydrogène, un radical alkyle en $C_1$-$C_4$, un radical hydroxyalkyle en $C_1$-$C_4$ et à l'exception des composés de formules suivantes :

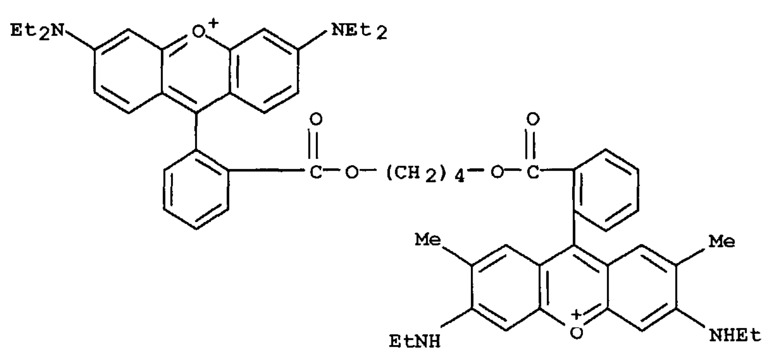

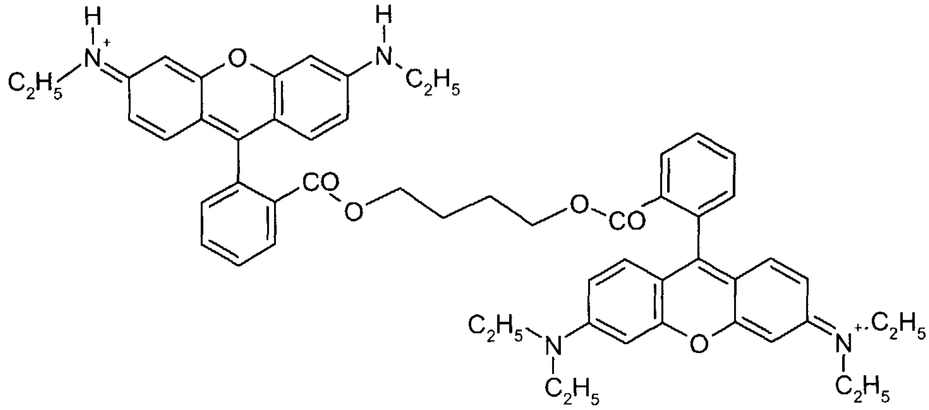

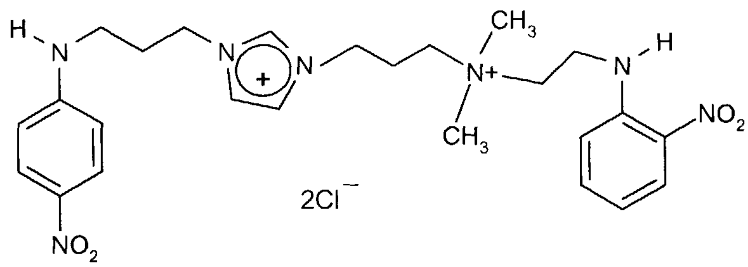

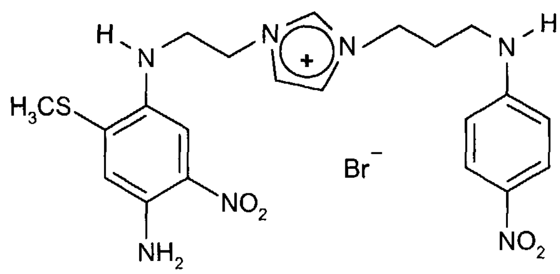

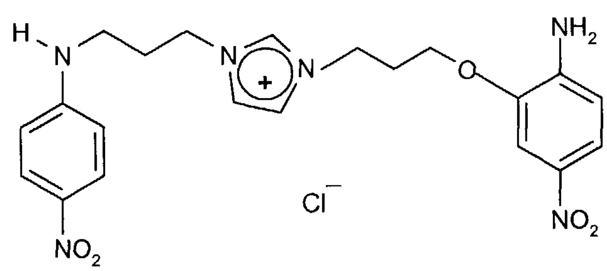

On a constaté que la composition tinctoriale selon l'invention permettait d'obtenir des couleurs puissantes, stables à la lumière, résistantes aux intempéries, aux lavages et à la transpiration, et tenaces dans le temps.

Mais d'autres caractéristiques et avantages de la présente invention apparaîtront plus clairement à la lecture de la description et des exemples qui vont être présentés.

Dans la suite, et à moins d'une indication différente, on entend par radical alkyle substitué, aryle (ou aromatique) substitué ou hétéroaryle (ou hétéroaromatique) substitué, un radical alkyle, aryle ou hétéroaryle portant un ou plusieurs radicaux choisis parmi un radical hydroxyle ; un atome d'halogène, et de préférence le chlore, le fluor ; un radical alcoxy en $C_1$-$C_8$ linéaire ou ramifié, substitué ou non, de préférence en $C_1$-$C_4$ ; un radical monohydroxy alcoxy dont la partie alkyle est en $C_1$-$C_8$, de préférence en $C_1$-$C_4$, linéaire ou ramifiée, substituée ou non ; un radical polyhydroxyalcoxy, en $C_2$-$C_8$, de préférence en $C_2$-$C_4$, linéaire ou ramifié, substitué ou non ; un radical amino substitué ou non par un ou plusieurs radicaux alkyle, identiques ou différents, en $C_1$-$C_8$, de préférence en $C_1$-$C_6$, linéaires ou

ramifiés, substitués ou non et/ou par un ou plusieurs radicaux aryles, de préférence en $C_6$, éventuellement substitués ; un radical thiol ; un radical alkylthio en Ci-$C_8$, de préférence en $C_1$-$C_4$, linéaire ou ramifiée, substitué ou non ; un radical carboxylique sous forme acide ou salifiée (de préférence avec un métal alcalin ou un ammonium, substitué ou non) ; un radical alcoxycarbonyle dont la partie alkyle est en $C_1$-$C_8$, de préférence en $C_1$-$C_4$, linéaire ou ramifiée, substituée ou non ; un radical alkylamide dont la partie alkyle est en $C_1$-$C_8$, de préférence en $C_1$-$C_4$, linéaire ou ramifié, substitué ou non ; un radical alkylcarbamyle dont la partie alkyle est en $C_1$-$C_8$, de préférence en $C_1$-$C_4$, linéaire ou ramifiée, substituée ou non ; un radical nitro ; un radical sulfonyle ; un radical alkylsulfonyle en $C_1$-$C_8$, de préférence en $C_1$-$C_4$, linéaire ou ramifié substitué ou non ; un radical sulfonylamino ; un radical alkylsulfonylamido dont la partie alkyle est en $C_1$-$C_8$, de préférence en $C_1$-$C_4$, linéaire ou ramifiée, substituée ou non.

Rappelons qu'un radical hétéroaromatique, ou hétéroaryle, correspond à un radical aromatique dans lequel au moins l'un des atomes de carbone est remplacé par un hétéroatome, et plus particulièrement, l'azote, l'oxygène ou le soufre.

De plus, lorsqu'il est indiqué que le radical alkyle ou aryle ou la partie alkyle ou aryle d'un radical substituant un autre radical, est lui-même substitué, on entend qu'il comprend un ou plusieurs substituants choisis parmi les groupements hydroxyle ; amino ; amino substitués par un ou plusieurs radicaux alkyle, identiques ou non, linéaires ou ramifiés, en $C_1$-$C_4$, éventuellement porteurs d'au moins un groupement hydroxyle ; des radicaux alcoxy, linéaires ou ramifiés en $C_1$-$C_4$, éventuellement porteurs d'au moins un ou plusieurs groupements hydroxyle.

Lorsqu'il est fait mention de radicaux amino portant deux substituants choisis parmi des radicaux alkyle éventuellement substitué, il est entendu que lesdits radicaux alkyle peuvent aussi former avec l'atome d'azote auquel ils sont liés, un cycle à 5 ou 6 chaînons dont l'un au moins des atomes de carbone peut être remplacé par un ou plusieurs atomes d'azote, d'oxygène ou de soufre.

De plus, sauf indication contraire, les bornes délimitant l'étendue d'une plage de valeurs sont comprises dans cette plage de valeurs.

En outre, les colorants mixtes selon l'invention étant cationiques, leur(s) contre-ion(s) sont choisis parmi les anions cosmétiquement acceptables, de nature minérale ou oragnique. A titre d'exemples d'anions de nature minérale, on peut citer notamment les halogénures, comme les chlorures, les bromures ; les hydroxydes ; les sulfates ; les hydrogénosulfates ; les carbonates, les hydrogénocarbonates.

A titre d'exemples d'anions de nature organique, conviennent les anions tels que l'acétate ; le citrate ; le tartrate ; les alkylsulfates pour lesquels la partie alkyle, linéaire ou ramifiée, est en $C_1$-$C_6$, comme l'ion méthosulfate, éthosulfate ; les alkylsulfonates pour lesquels la partie alkyle, linéaire ou ramifiée, est en $C_1$-$C_6$ ; les arylsulfonates pour lesquels la partie aryle, de préférence phényle, est éventuellement substituée par un ou plusieurs radicaux alkyle en $C_1$-$C_4$.

Les colorants mixtes présents dans la composition selon l'invention vont tout d'abord être décrits.

Comme indiqué précédemment, les colorants mixtes comprennent au moins deux chromophores différents ; au moins l'un des chromophores est choisi parmi les chromophores de la famille des composés azines cycliques et/ou nitré (hétéro)aromatique éventuellement associé à au moins un chromophore de la famille des méthines et/ou des carbonyles ; les chromophores étant liés entre eux par l'intermédiaire d'au moins un bras de liaison stoppant la délocalisation des électrons de chacun des chromophores.

De préférence l'un au moins des chromophores du colorant mixte porte une ou plusieurs charges cationiques.

Par chromophore, on entend un radical issu d'un colorant, c'est-à-dire un radical d'une molécule absorbant dans le domaine visible entre 400 et 800 nm. Il est à noter de plus que cette absorbance du colorant ne nécessite ni oxydation préalable de celui-ci, ni association avec d'autre(s) espèce(s) chimique(s).

Lorsqu'il est précisé que les chromophores sont différents, cela signifie qu'au moins deux d'entre eux, de préférence tous, diffèrent par leur structure chimique. De tels chromophores peuvent être des chromophores issus de familles différentes ou d'une même famille à la condition de présenter des structures chimiques différentes. Par exemple, les chromophores peuvent être choisis dans une même famille de colorants mais différer par la structure chimique des radicaux les constituant.

Selon un mode de réalisation particulier de l'invention, le colorant mixte comprend deux à quatre chromophores, avantageusement deux à trois chromophores.

Il est à noter que lorsque le colorant comprend plus de deux chromophores, au moins l'un de ces chromophores est différent de l'autre ou des autres.

Conformément à un mode de réalisation préféré de l'invention, le colorant mixte comprend deux chromophores.

Conformément à une variante particulière de l'invention, le ou les chromophores cationiques sont des chromophores comprenant au moins un atome d'azote quaternisé.

De plus, la ou les charges cationiques peuvent être engagées ou non dans un cycle.

Par ailleurs, comme indiqué plus haut, au moins l'un des chromophores du colorant mixte présente au moins une charge cationique, et plus particulièrement une seule charge cationique.

De préférence, selon cette variante, chacun des chromophores comprend au moins une charge cationique, et de manière très avantageuse, une seule charge cationique.

Conformément à un mode de réalisation particulier de l'invention, le colorant mixte possède une charge globale cationique, dans les conditions d'utilisation de ce colorant mixte.

Comme indiqué auparavant, le colorant mixte de la présente invention peut comprendre au moins un chromophore de la famille des azines cycliques.

Plus particulièrement, le chromophore de la famille des azines cycliques est choisi parmi les radicaux issus des colorants choisi parmi les azine, xanthène, thioxanthène, fluorindine, acridine, (di)oxazine, (di)thiazine, pyronine.

Parmi les chromophores de la famille des azines cycliques, on peut citer les composés de formule suivante (I) ainsi que ses formes tautomères :

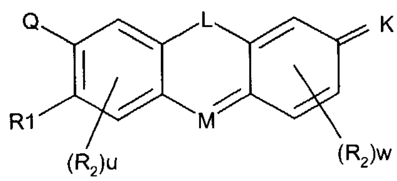

formule dans laquelle :

L, représente un hétéroatome de préférence choisi parmi l'oxygène, le soufre ; un groupe NH ; un groupe N-$R_3$,
M représente un hétéroatome de préférence choisi parmi l'oxygène, le soufre, l'azote ; un groupe $N^+$-$R_3$ ; un groupe CH ; un groupe C-$R_4$ ;
Q, K, identiques ou non, représentent un groupement hydroxyle ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle, un radical aryle éventuellement substitué, un radical alkyl($C_1$-$C_8$)aryle éventuellement substitué ; un groupement ammonium de type N+$(R_5)_t$ avec t égal à 2 pour K et à 3 pour Q ; $R_5$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl($C_1$-$C_8$) aryle la partie aryle étant éventuellement substituée ; un radical alkyle en $C_1$-$C_8$, linéaire ou ramifiée, éventuellement substituée, un radical alcoxy, linéaire ou ramifié, en $C_1$-$C_8$, éventuellement substitué ;
A la condition que Q et K ne représentent pas à la fois un groupement ammonium de type $N^+(R_5)_t$ ;
$R_1$, $R_2$, identiques ou non, représentent un atome d'hydrogène ; un radical alkyle, linéaire ou ramifié, en $C_1$-$C_8$ éventuellement substitué ; un radical amino ; un radical amino substitué ou non par un ou plusieurs radicaux, identiques ou non, choisis parmi les radicaux alkyle, linéaires ou ramifiés, en $C_1$-$C_8$ éventuellement substitués, les radicaux phényle éventuellement substitués ; un atome d'halogène de préférence le chlore, le fluor ;
Au cas où Q représente un radical amino substitué ou non, ou un groupement hydroxyle, $R_1$ peut représenter un radical alkylamino ou alcoxy formant avec l'atome d'azote ou d'oxygène du radical **J**, un cycle à 6 chaînons, éventuellement condensé avec un radical aromatique, ledit radical aromatique étant éventuellement substitué par au moins un groupement amino ou amino substitué ou non par un ou plusieurs radicaux identiques ou non, choisis parmi les radicaux alkyle, linéaires ou ramifiés, en $C_1$-$C_8$ éventuellement substitués, les radicaux phényle éventuellement substitués ;
$R_3$, $R_4$, identiques ou non, représentent un radical alkyle, linéaire ou ramifié en $C_1$-$C_8$ éventuellement substitué, de préférence par au moins un hydroxyle ; au moins un radical alcoxy, linéaire ou ramifié en $C_1$-$C_8$ ; au moins un radical amino ; au moins un radical amino substitué par un ou plusieurs radicaux alkyles, identiques ou non, linéaires ou ramifiés, en $C_1$-$C_8$, éventuellement porteurs d'au moins un radical hydroxyle ;
$R_3$, $R_4$, identiques ou non, peuvent aussi représenter un radical aryle, de préférence en $C_6$, éventuellement substitué, de préférence par au moins un radical alkyle, linéaire ou ramifié, en $C_1$-$C_8$ ; par au moins un groupement hydroxyle ; par au moins un radical alcoxy, linéaire ou ramifié, en $C_1$-$C_8$ ; par au moins un radical amino ; par au moins un radical amino substitué par un ou plusieurs radicaux alkyle, identiques ou non, linéaires ou ramifiés, en $C_1$-$C_8$, éventuellement porteurs d'au moins un groupement hydroxyle ; un atome d'halogène et de préférence le chlore ou le fluor ; par au moins un groupement nitro ; par au moins un groupement cyano. De préférence $R_3$ et $R_4$, identiques ou non, représentent un radical alkyle, linéaire en $C_1$-$C_4$, un radical aryle éventuellement substitué ;
Le coefficient u est égal à 2 ; w est égal à 3.

Par ailleurs, le chromophore est relié plus particulièrement au bras de liaison par l'intermédiaire de l'un des

radicaux $R_1$, $R_2$, $R_3$, $R_4$, $R_5$, ou par l'intermédiaire d'un cycle. Dans ce cas, le radical porté par le cycle représente une liaison simple entre le chromophore et le bras de liaison.

Conformément à un mode de réalisation préféré, le chromophore de la famille des azines cycliques est représenté par les formules ci-dessous, ainsi que, le cas échéant, leurs formes tautomères :

| | |
|---|---|
| Xanthènes | 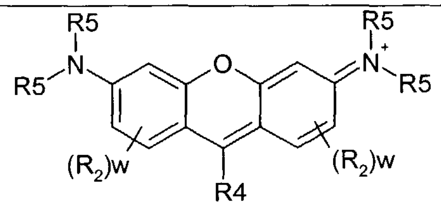 |
| Thioxanthènes | 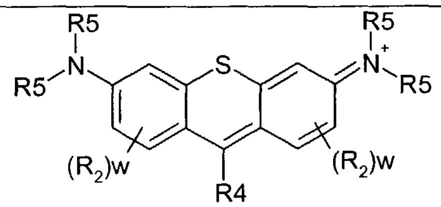 |
| Acridines | 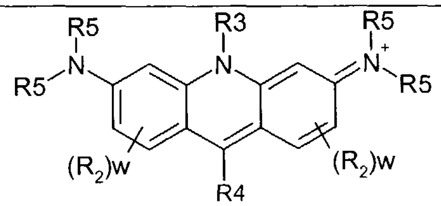 |
| Oxazines | 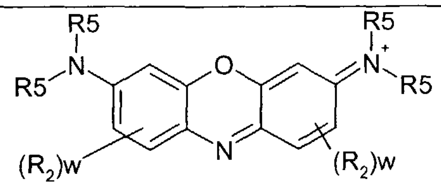 |

| | |
|---|---|
| Dioxazines | 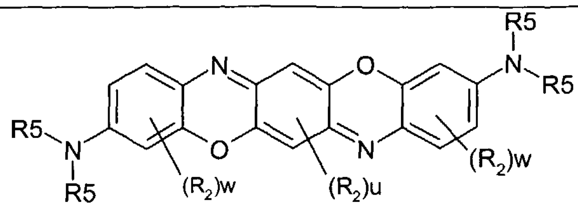 |
| Thiazines | 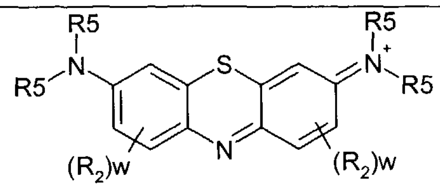 |
| Phénazines | 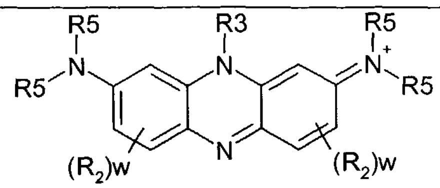 |

Formules dans lesquelles les radicaux et les coefficients u et w ont été définis précédemment.

Le chromophore est de plus relié au bras de liaison par l'intermédiaire de l'un des radicaux $R_2$, $R_3$, $R_4$, $R_5$ ou par l'intermédiaire d'un cycle. Dans ce cas, le radical porté par le cycle représente une liaison simple entre le chromophore et le bras de liaison.

A titre de chromophore possible, le colorant mixte peut comprendre au moins un chromophore de la famille des composés nitrés (hétéro)aromatiques.

Plus particulièrement, on peut citer par exemple les composés correspondant aux formules (II) et (III) ci-dessous, ainsi que leurs formes tautomères :

| | |
|---|---|
| benzénique | 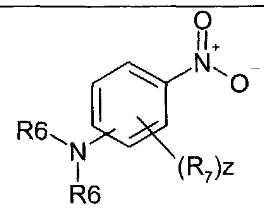 (II) |
| Pyridinique | 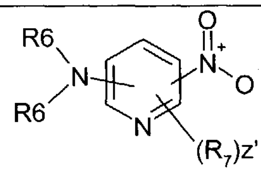 (III) |

Formules dans lesquelles

$R_6$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl($C_1$-$C_8$)aryle la partie aryle étant éventuellement substituée ; un radical alkyle en $C_1$-$C_8$, linéaire ou ramifiée, éventuellement substituée, un radical alcoxy, linéaire ou ramifié, en $C_1$-$C_8$, éventuellement substitué ;

$R_7$, identiques ou différents, représentent un atome d'hydrogène ; alkyle linéaire ou ramifié en $C_1$-$C_4$, éventuellement substitué ; un radical alcoxy linéaire ou ramifié en $C_1$-$C_8$, éventuellement substitué ; un radical aryle en $C_6$ éventuellement substitué ; un radical amino ; un radical amino substitué par un ou plusieurs radicaux choisis parmi les radicaux alkyle, identiques ou non, linéaires ou ramifiés, en $C_1$-$C_8$, de préférence en $C_1$-$C_4$, éventuellement substitués, de préférence par au moins un groupe hydroxyle, alcoxy, linéaire ou ramifié en $C_1$-$C_4$, thioalkyl linéaires ou ramifiés en $C_1$-$C_4$, alkylsulfonamido linéaires ou ramifiés en $C_1$-$C_4$, parmi les radicaux aryle en $C_6$ éventuellement substitué et parmi les radicaux hétéroaryle comprenant 5 à 6 chaînons éventuellement substitués ; un groupe hydroxyle ; un groupe nitro ; un groupe cyano ;

z est égal à 4 ; z'est égal à 3.

En outre, le chromophore est relié au bras de liaison par l'intermédiaire de $R_6$, $R_7$ ou par l'intermédiaire du cycle. Dans ce cas, le radical porté par le cycle représente une liaison simple entre le chromophore et le bras de liaison.

Comme indiqué auparavant, le colorant mixte selon la présente invention peut comprendre au moins un chromophore de la famille des méthines.

Ces derniers sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle.

Plus particulièrement, les chromophores de cette famille sont issus de chromophores de type méthine, azométhine, mono- et di- arylméthane, indamines (ou diphénylamines), indophénols, indoanilines, carbocyanines, azacabocyanines et leurs isomères, diazacarbocyanines et leurs isomères, tétraazacarbocyanines, hémicyanines.

Plus particulièrement, parmi les chromophores de la famille des méthines, on peut citer les chromophores correspondant à la formule suivante (IV) ainsi que ses formes tautomères :

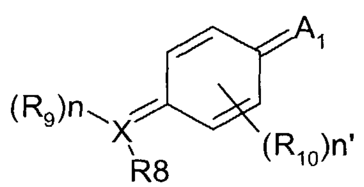

Formule dans laquelle :

- $R_8$, $R_9$, identiques ou non, représentent un atome d'hydrogène ; un radical aryle en $C_6$-$C_{30}$ un radical alkyl($C_1$-$C_8$) aryle, la partie aryle étant éventuellement substituée par un ou plusieurs groupements, identiques ou non, choisis de préférence parmi les groupements hydroxyle, alcoxy, linéaire ou ramifié, substitué ou non, en $C_1$-$C_4$, amino, amino substitué par un ou plusieurs radicaux alkyles, identiques ou non, linéaires ou ramifiés, en $C_1$-$C_4$ éventuellement porteurs d'au moins un groupement hydroxyle, les atomes d'halogène, de préférence le chlore ; un radical hétérocyclique, choisi notamment parmi les hétérocycles thiophène, furane, pipéronyle, indole, indoline, pyridine, carbazole, déhydroquinoléïne, chromone ;
- $R_8$, $R_9$, ne peuvent en outre représenter simultanément ni un radical aromatique, ni un radical hétéroaromatique ;
- $R_{10}$, identiques ou différents, représentent un atome d'hydrogène ; un radical alkyle, linéaire ou ramifié, en $C_1$-$C_8$ éventuellement substitué ; un radical aryle en $C_6$-$C_{30}$, éventuellement substitué ; un radical amino ; un radical amino substitué par ou plusieurs radicaux alkyle, linéaires ou ramifiés, en $C_1$-$C_4$, éventuellement porteurs d'au moins un groupement hydroxyle ; un groupement hydroxyle ; un radical alcoxy, linéaire ou ramifié, en $C_1$-$C_8$, éventuellement porteur d'au moins un groupement hydroxyle, alcoxy en $C_1$-$C_4$ ; un atome d'halogène et de préférence le chlore ;
- X représente un atome d'azote, un atome de carbone ;
- le coefficient n représente 0 lorsque X est un atome d'azote, et 1 lorsque X est un atome de carbone ;
- le coefficient n' est égal à 4 ;
- $A_1$ représente un groupement amino ; un groupement amino substitué par un ou plusieurs radicaux alkyles en $C_1$-$C_8$, identiques ou non, linéaires ou ramifiés, éventuellement porteurs d'au moins un groupement hydroxyle, un groupement ammonium $N^+(R_{11})_2$ ; $R_{11}$, identiques ou non, représentent un radical alkyle en $C_1$-$C_8$ éventuellement substitué ; un radical aryle en $C_6$ éventuellement substitué, de préférence par un ou plusieurs hydroxyle, atomes d'halogène, de préférence le chlore, le fluor, groupes nitro, groupes cyano, alcoxy, linéaires ou ramifiés, en $C_1$-$C_4$, monohydroxy alcoxy en $C_1$-$C_4$, linéaires ou ramifiés, polyhydroxyalcoxy, en $C_2$-$C_4$, linéaires ou ramifiés, amino substitués ou non par un ou plusieurs radicaux alkyle, hydroxyalkyle, identiques ou différents, en $C_1$-$C_4$, linéaires ou ramifiés.

Par ailleurs, le chromophore est relié au bras de liaison par l'intermédiaire du groupe $A_1$ ou de l'un des radicaux $R_8$, $R_9$, $R_{10}$ et $R_{11}$ ou directement sur le ou les cycles (hétéro)aromatique. Dans ce dernier cas, le radical $R_{10}$ concerné représente alors une liaison simple entre le chromophore et le bras de liaison.

De préférence, lorsque $R_8$ ou $R_9$ représente un radical aryle en $C_6$, ce dernier peut éventuellement être substitué, de préférence par au moins un groupement hydroxyle, au moins un groupement amino ; au moins un groupement amino substitué par un ou plusieurs radicaux alkyles linéaires ou ramifiés, en $C_1$-$C_8$, identiques ou non, éventuellement porteurs d'au moins un groupement hydroxyle ; un ou plusieurs atomes d'halogène ; au moins un radical alkylsulfonamido linéaire ou ramifié, en $C_1$-$C_{12}$ (alkyl-$SO_2$-NH-) ; au moins un radical alkylsulfamoyle linéaire ou ramifié, en $C_1$-$C_{12}$ (alkyl-NH-$SO_2$-) au moins un radical acyloxy dont la partie alkyle linéaire ou ramifiée, est en en $C_1$-$C_{12}$ ; alcoxycarbonyle dont la partie alkyle linéaire ou ramifiée est en $C_1$-$C_{12}$ ; au moins un radical carboxyle.

Conviennent aussi les chromophores de formule suivante (V) :

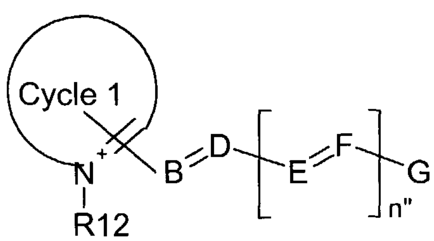

et le cas échéant leurs formes tautomères ;
formule dans laquelle

B, D, E, et F, identiques ou différents, représentant un atome d'azote ou un groupe C-$R_{13}$,
$R_{13}$, identiques ou non, représentent un atome d'hydrogène, un radical alkyle en $C_1$-$C_8$ éventuellement substitué, de préférence par au moins un hydroxyle ; un radical alcoxy, linéaire ou ramifié, en $C_1$-$C_4$ ; un radical amino ; un radical amino substitué par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué, un radical hétéroaryle comprenant de 5 à 12 chaînons, éventuellement substitué ;
n" = 0 ou 1 ;

G représentant un Cycle 4, ou les restes :

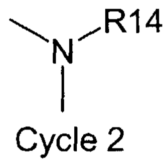

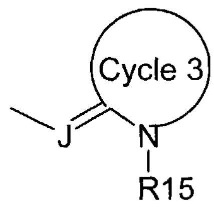

formules dans lesquelles :

$R_{12}$ et $R_{15}$ représentent indépendamment l'un de l'autre, un radical alkyle, linéaire ou ramifié, en $C_1$-$C_8$ éventuellement substitué ; un radical benzyle éventuellement substitué ;

$R_{14}$ représente un atome d'hydrogène, un radical alkyle en $C_1$-$C_8$ éventuellement substitué, de préférence par au moins un hydroxyle, un radical alcoxy, linéaire ou ramifié, en $C_1$-$C_4$, un radical amino, un radical amino substitué par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué ; un radical hétéroaryle en $C_2$-$C_{12}$ éventuellement substitué ;

**J** représente un atome d'azote ou un groupe C-$R_{16}$ avec $R_{16}$ ayant la même signification que $R_{13}$ ;

**Cycle 1** étant choisi parmi les radicaux hétéroaromatiques à 5 à 12 chaînons, portant au moins une charge cationique sur un atome d'azote et comprenant éventuellement au moins un autre hétéroatome choisi parmi l'azote, l'oxygène et/ou le soufre ; lesdits radicaux étant éventuellement substitués par au moins un radical alkyle, linéaire ou ramifié, en $C_1$-$C_8$ substitué ou non ; au moins un radical alcoxy, linéaire ou ramifié, en $C_1$-$C_8$ substitué ou non ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; au moins un radical alcoxycarbonyle, linéaire ou ramifié, en $C_1$-$C_8$ ; au moins un nitro ; au moins un cyano ; au moins un radical alkylsulfonamido linéaire ou ramifié, en $C_1$-$C_{12}$ (alkyl-$SO_2$-NH-) ; alkylsulfamoyle linéaire ou ramifié, en $C_1$-$C_{12}$ (alkyl-NH-$SO_2$-) ;

**Cycle 2** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons ; au moins un groupement hydroxyle. De préférence Cycle 2 représente un radical aromatique en $C_6$-$C_{30}$, éventuellement substitué comme indiqué ci-dessus ;

**Cycle 3** étant choisi parmi les radicaux hétéroaromatiques à 5 ou 6 chaînons comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; au moins un radical alcoxycarbonyle linéaire ou ramifié en $C_1$-$C_8$ ; nitro ; cyano ; au moins un radical alkylsulfonamido linéaire ou ramifié en $C_1$-$C_{12}$ (alkyl-$SO_2$-NH-) ; au moins un radical alkylsulfamoyle linéaire ou ramifié en $C_1$-$C_{12}$ (alkyl-NH-$SO_2$-) ;

**Cycle 4** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons; au moins un groupement hydroxyle ;

à la condition que lorsque n'' vaut 1 et G représente un cycle, alors B, D, E et F ne peuvent représenter simultanément un atome d'azote ; et que lorsque n'' vaut 0 et G représente un cycle, alors B et D ne représentent pas simultanément un atome d'azote.

Par ailleurs, le chromophore est relié au bras de liaison par l'intermédiaire de l'un des radicaux $R_{12}$, $R_{13}$, $R_{14}$,

$R_{15}$, et $R_{16}$ ou des cycles. Dans ce cas, le radical porté par le cycle représente une liaison simple entre le chromophore et le bras de liaison.

La formule (V) comprend les isomères de position correspondant aux différentes possibilités d'implantation de la liaison de B sur le cycle 1 par rapport à l'atome d'azote quaternisé).

Selon un mode de réalisation particulièrement avantageux de l'invention, le chromophore de la famille des méthines est choisi parmi les composés de formules suivantes, ainsi que leurs formes tautomères :

| | |
|---|---|
| Indamines (Diphénylamines) | 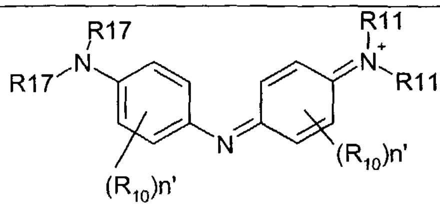 |
| Diphénylméthanes | 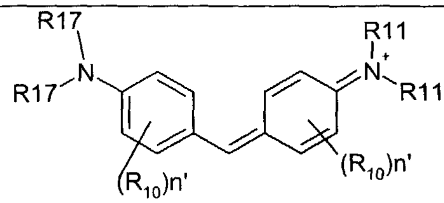 |
| Indoaniline | 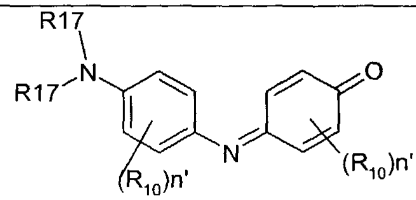 |
| Indophénol | 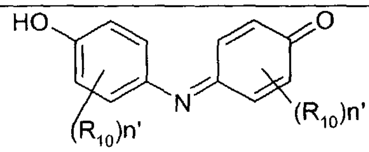 |
| Carbocyanine | 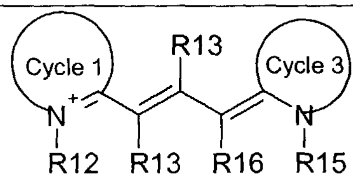 |
| Azacarbocyanine | 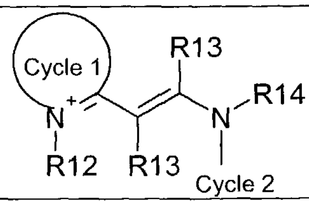 |
| Isomère azacarbocyanine | 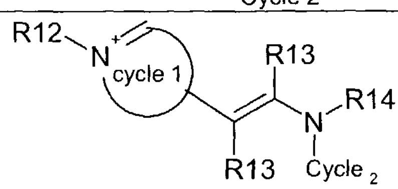 |
| Diazacarbocyanine | 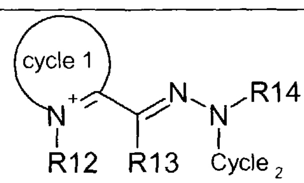 |

| | |
|---|---|
| Isomère diazacarbocyanine | 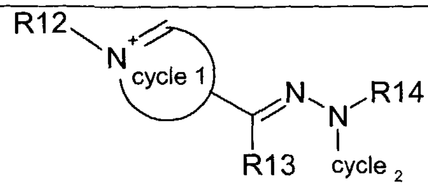 |
| Tétraazacarbocyanine | 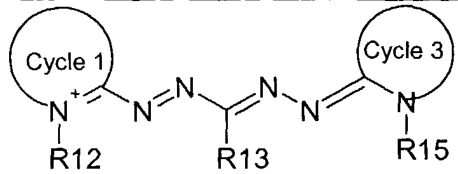 |
| Hémicyanine | 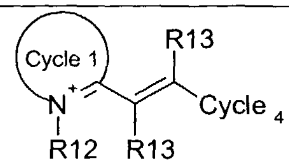 |

Formules dans lesquelles :

**R**$_{17}$ identiques ou non, représentent un atome d'hydrogène, un radical alkyle en $C_1$-$C_8$ éventuellement substitué ; un radical aryle en $C_6$ éventuellement substitué, de préférence par un ou plusieurs hydroxyle, atomes d'halogène, de préférence le chlore, le fluor, groupes nitro, groupes cyano, alcoxy, linéaires ou ramifiés, en $C_1$-$C_4$, monohydroxy alcoxy en $C_1$-$C_4$, linéaires ou ramifiés, polyhydroxyalcoxy, en $C_2$-$C_4$, linéaires ou ramifiés, amino substitués ou non par un ou plusieurs radicaux alkyle, hydroxyalkyle, identiques ou différents, en $C_1$-$C_4$, linéaires ou ramifiés ;
Les groupements et radicaux $R_{11}$, $R_{12}$, $R_{13}$, $R_{14}$, $R_{15}$, $R_{16}$, $Cycle_1$, $Cycle_2$, $Cycle_3$ et $Cycle_4$ étant définis comme précédemment.
De préférence, le groupement $Cycle_1$ représente un cycle imidazolium, pyridinium ou indolinium, éventuellement substitué comme indiqué auparavant.
De préférence, le groupement $Cycle_3$ représente un cycle imidazole, pyridine or indoline, éventuellement substitué comme indiqué auparavant.
De préférence, le groupement $Cycle_2$ représente un radical aromatique en $C_6$ éventuellement substitué comme indiqué auparavant.
De préférence, le groupement $Cycle_4$ représente un radical aromatique en $C_6$ éventuellement substitué comme indiqué auparavant.

Précisons que le chromophore peut être relié au bras de liaison par l'intermédiaire de l'un des groupes $R_{10}$, $R_{11}$, $R_{12}$, $R_{13}$, $R_{14}$, $R_{15}$ et $R_{16}$ ou par l'intermédiaire d'un cycle aromatique ou hétéroaromatique. Dans le cas de rattachement par l'intermédiaire d'un cycle, le radical porté sur ledit cycle représente une liaison simple entre le chromophore et le bras de liaison.

Conformément à un mode de réalisation particulier de l'invention, le chromophore est choisi parmi les composés de formule (V) et de préférence parmi les diazacarbocynaines et leurs isomères, les hémicyanines. Plus particulièrement, $R_{13}$, identiques ou non, représentent un atome d'hydrogène, un radical alkyle en $C_1$-$C_8$ éventuellement substitué, de préférence par au moins un hydroxyle ; un radical aryle en $C_6$ éventuellement substitué ; n = 0 ; G représentant un Cycle4, ou -N($R_{14}$)-Cycle2 avec $R_{14}$ représentant un atome d'hydrogène, un radical alkyle en $C_1$-$C_8$ éventuellement substitué, de préférence par au moins un hydroxyle ; les cycle2 et cycle4, identiques u non, représentant un radical aromatique en $C_6$, éventuellement substitués.

Selon ce mode de réalisation particulier de l'invention, le chromophore et le bras de liaison sont reliés par l'intermédiaire du radical $R_{12}$.

Il est à noter que les chromophores décrits ci-dessus peuvent être préparés selon l'enseignement des brevets et demandes de brevets suivants GB 822846 ; DE 1254118 ; GB 1047796 ; US 3652556 ; US 3423427 ; BE 702239 ; GB 702240 ; US 3995088 ; US 4054718 ; US 4670385 ; US 5094688 ; US 5097034.

Le colorant mixte peut de même comprendre au moins un chromophore de la famille des carbonyles.

Parmi les chromophores de ce type, on peut citer par exemple les chromophores issus des colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone,

isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Plus particulièrement, le chromophore de la famille des carbonyles est représenté par la formule suivante (VI) :

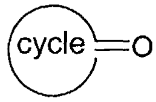

Formule dans laquelle le cycle représente un cycle à 5 ou 6 chaînons, dont éventuellement au moins l'un d'entre eux est remplacé par un hétéroatome choisi parmi l'oxygène, l'azote, le soufre, ou par une fonction carbonyle additionnelle ; ledit cycle étant éventuellement substitué par un radical ou plusieurs radicaux choisis parmi les radicaux alkyle, linéaire ou ramifié, en $C_1$-$C_8$, éventuellement substitués ; par un ou plusieurs radicaux hydroxyle ; les atomes d'halogène comme de préférence le chlore ; les groupements nitro, cyano, amino, alkylamino ; ledit cycle étant éventuellement condensé avec un ou plusieurs cycles aromatiques en $C_6$, ce ou ces cycles pouvant eux-mêmes être condensés à un ou plusieurs cycles aromatiques dont éventuellement au moins l'un des atomes de carbone est remplacé par un ou plusieurs hétéroatomes choisis parmi l'oxygène, l'azote ou le soufre.

Par ailleurs, le chromophore étant relié au bras de liaison par l'intermédiaire de l'un des radicaux substituants les cycles ou par l'intermédiaire d'un cycle. Dans ce dernier cas, le radical porté par le cycle représente une liaison simple entre le chromophore et le bras de liaison.

Conformément à un mode de réalisation préféré de l'invention, le chromophore de la famille des carbonyles est représenté par l'une des formules suivantes, ainsi que les formules tautomères :

| | |
|---|---|
| Acridones | 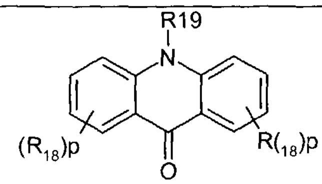 |
| Anthraquinones | 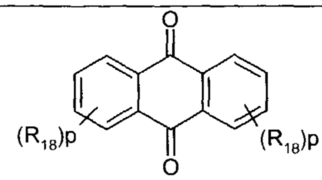 |
| Benzanthrones | 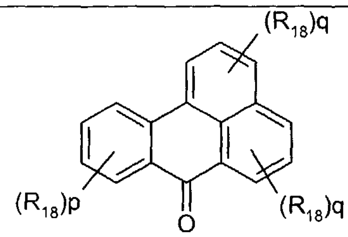 |
| Benzoquinones | 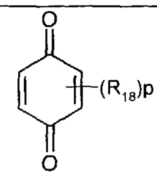 |
| Flavones | 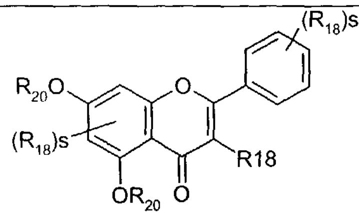 |
| Indanthrones | 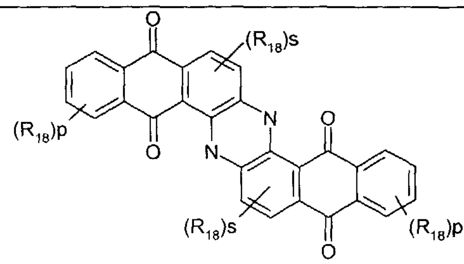 |
| Naphthoquinones | 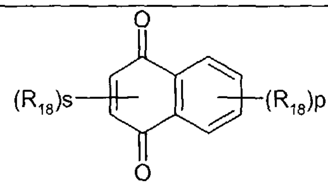 |
| Quinacridones | 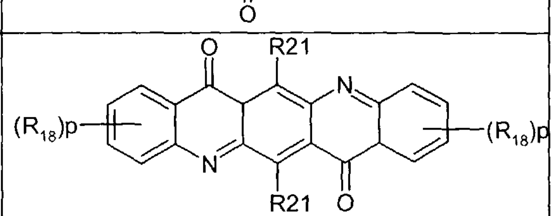 |

| | |
|---|---|
| Indigoïdes | 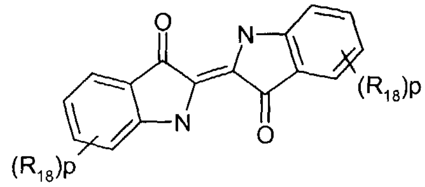 |
| Thioindigos | 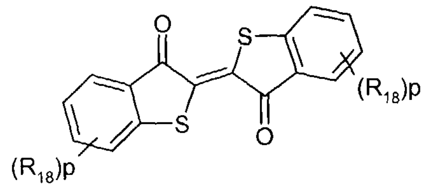 |
| Naphthalimides | 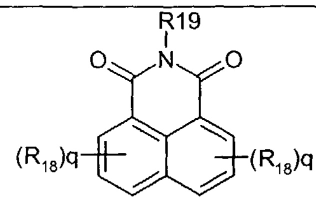 |
| Dicétopyrrolopyrroles | 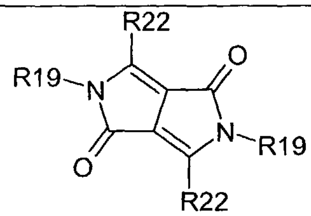 |
| Coumarines | 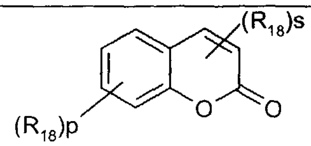 |

Formules dans lesquelles :

$R_{18}$, $R_{19}$, $R_{21}$, identiques ou non, représentent un atome d'hydrogène ; un radical alkyle, linéaire ou ramifié, en $C_1$-$C_8$, éventuellement substitué ; un groupement hydroxyle ; un radical alcoxy, linéaire ou ramifié, en $C_1$-$C_8$ ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle ; un atome d'halogène et de préférence le chlore ou le fluor ; un groupement nitro ; un groupement cyano ;
$R_{20}$ représente un atome d'hydrogène, un radical alkyle, linéaire ou ramifié, en $C_1$-$C_8$ ; éventuellement substitué ;
$R_{22}$, identiques ou non, représentent un radical aryle en $C_6$, éventuellement substitué, de préférence par au moins un hydroxyle, au moins un amino, au moins un amino substitué par un ou plusieurs radicaux alkyles, identiques ou non, linéaires ou ramifiés en $C_1$-$C_8$ éventuellement porteurs d'au moins un hydroxyle, au moins un groupe alcoxy, linéaire ou ramifié, en $C_1$-$C_8$ éventuellement porteur d'au moins un hydroxyle, au moins un atome d'halogène de préférence le chlore, le fluor, au moins un groupement nitro, au moins un groupement cyano ;
p est égal à 4 ; q est égal à 3 ; r est égal à 5; s est égal à 2.

Le chromophore peut être relié au bras de liaison par l'intermédiaire de l'un des radicaux $R_{18}$, $R_{19}$, $R_{20}$, $R_{21}$, $R_{22}$ ou par l'intermédiaire de l'un des cycles aromatiques ou hétéroaromatiques. Dans ce cas, le radical porté par le cycle représente une liaison simple entre le chromophore et le bras de liaison.

Ainsi, à titre d'exemples de colorants mixtes comprenant deux chromophores, on peut citer par exemple les colorants mixtes suivants :

Azine - bras de liaison - azine
Azine - bras de liaison - méthine
Azine - bras de liaison - carbonyle
Azine - bras de liaison - nitré

Nitré - bras de liaison - nitré
Nitré - bras de liaison - méthine
Nitré - bras de liaison - carbonyle

Comme indiqué précédemment, les chromophores du colorant mixte sont reliés entre eux au moyen d'au moins un bras de liaison stoppant la délocalisation des électrons de chacun des chromophores.

Ainsi, le bras de liaison comprend un atome ou un groupe d'atomes qui isole(nt) chacun des chromophores du colorant mixte.

De préférence, la liaison entre le radical et le bras de liaison est réalisée par l'intermédiaire d'un atome d'azote ou d'oxygène.

Il est à noter en outre, que le bras de liaison peut être cationique ou non cationique.

De plus, le bras de liaison peut être divalent, trivalent ou tétravalent.

Selon un mode de réalisation avantageux de l'invention, le bras de liaison est choisi parmi une chaîne hydrocarbonée en $C_1$-$C_{20}$, de préférence en $C_1$-$C_{12}$, linéaire ou ramifiée, de préférence une chaîne alkyle, un ou plusieurs des atomes de carbone de ladite chaîne pouvant chacun être remplacés par un hétéroatome tel que le soufre, l'azote, l'oxygène, à la condition que la chaîne ne comprenne pas deux hétéroatomes adjacents ; par un hétérocycle comprenant 5 ou 6 chaînons, saturé ou non, comprenant de préférence au moins deux atomes d'azote ; ladite chaîne hydrocarbonée pouvant être insaturée ou contenir un radical arylène ; parmi un radical arylène ; parmi un radical divalent téréphtalamide ; parmi un radical divalent ou trivalent, par exemple de type triazine.

De préférence, le bras de liaison correspond à une chaîne alkyle en $C_1$-$C_{20}$, de préférence en $C_1$-$C_{12}$, linéaire ou ramifiée, dont au moins l'un des atomes de carbone peut être remplacé par un hétérocycle comprenant 5 ou 6 chaînons, saturé ou non, comprenant de préférence au moins deux atomes d'azote.

Les colorants mixtes de l'invention peuvent être préparés selon des réactions chimiques connues en soi, à partir de chromophores fonctionnalisés capables de réagir avec le bras de liaison choisi.

Par exemple, lorsque le bras de liaison est un groupe triazine alors le chromophore comporte un groupe réactif amino, OH ou SH, et la synthèse s'effectue, par exemple, selon les schémas ci dessous :

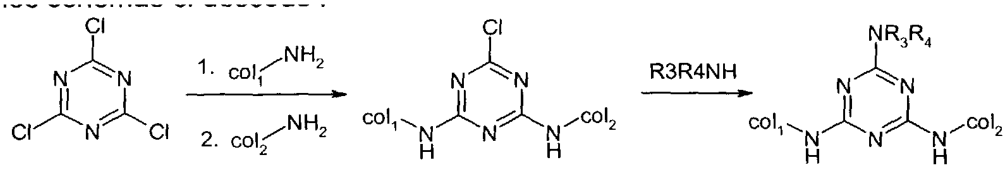


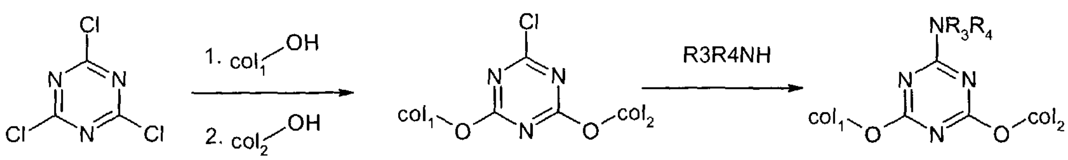


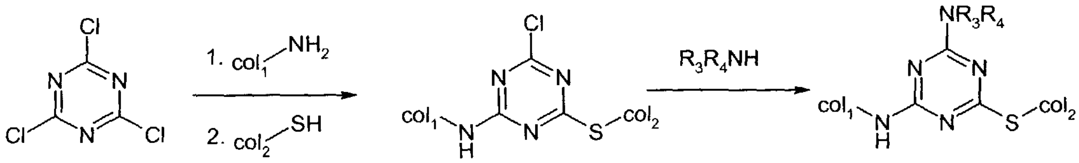


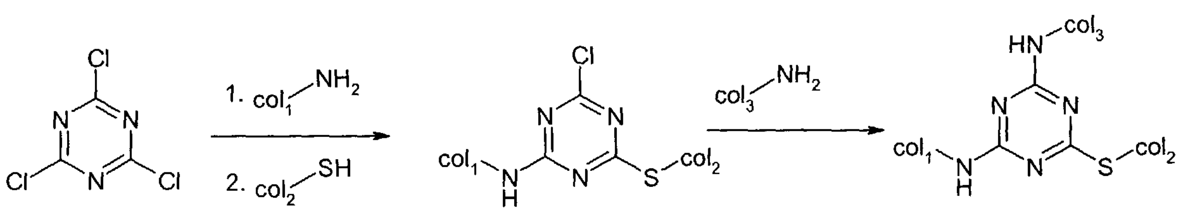

Selon une première étape, un premier chromophore est mélangé au composé susceptible de former le bras de liaison, par exemple la trichlorotriazine. Lorsque cette réaction est terminée, on ajoute au milieu réactionnel un

second chromophore. Cette séquence peut être répétée autant de fois qu'il y a de groupes réactifs sur le composé susceptible de former le bras de liaison.

Pour la préparation d'un colorant mixte Col1-bras liaison-Col 2, le rapport molaire du bras de liaison par rapport au colorant 1 est généralement compris entre 10:1 et 0,5:1, de préférence égal à 1:1. Ce rapport peut être modifié lorsqu'on utilise plus d'un bras de liaison ou plusieurs chromophores.

La température de réaction est en général comprise entre -100°C et +130°C, de préférence entre -5°C et 100°C. Le temps de réaction dépend de la réactivité des espèces en présence et de la température de réaction. En général, le temps de réaction est compris entre 10 minutes et 8 heures, de préférence entre 30 minutes et 4 heures.

Avantageusement, le pH du mélange réactionnel est compris entre 2 et 12.

La réaction peut en outre être conduite dans de l'eau et/ou dans un ou plusieurs solvants organiques.

Plusieurs publications décrivent la réaction d'association chimique entre deux chromophores identiques. On peut citer par exemple les documents ISBN 0901956759, WO 0278596, DE19845640, US 5708151.

En outre, les réactions ou les réactions d'un bras de liaison avec deux composés différents, colorants ou non, ont été décrites dans la littérature, par exemple dans WO03/029359, DE3335956, WO03/30909, WO0318021, Journal of Medicinal Chemistry 43(9), 2000, 1892-97 ; Chemiker Zeitung 117(7-8), 1987, 241-5.

Selon une autre possibilité, le colorant mixte peut être obtenu en suivant le schéma réactionnel suivant :

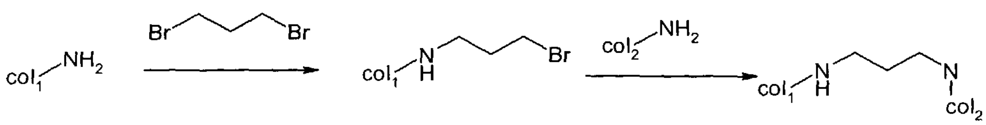

Selon une première étape, un premier chromophore est mélangé au composé susceptible de former le bras de liaison, par exemple le dibromopropane. Lorsque cette réaction est terminée, on ajoute au milieu réactionnel un second chromophore. Cette séquence peut être répétée autant de fois qu'il y a de groupes réactifs sur le composé susceptible de former le bras de liaison.

Pour la préparation d'un colorant mixte Col1-bras de liaison-Col 2, le rapport molaire du bras de liaison par rapport au colorant 1 est généralement compris entre 10:1 et 0,1:1, de préférence égal à 0,5:1. Ce rapport peut être modifié lorsqu'on utilise plus d'un bras de liaison ou plusieurs chromophores.

La température de réaction est en général comprise entre -100C et +130°C, de préférence entre -5°C et 100°C. Le temps de réaction dépend de la réactivité des espèces en présence et de la température de réaction. En général, le temps de réaction est compris entre 10 minutes et 24 heures, de préférence entre 30 minutes et 4 heures.

La réaction peut être conduite dans de l'eau et/ou dans un ou plusieurs solvants organiques.

Avantageusement, le pH du mélange réactionnel est compris entre 2 et 12.

La composition selon l'invention comprend habituellement une teneur en colorant mixte variant de 0,001 à 20% en poids, plus particulièrement, de 0,005 à 10% en poids, et de préférence, de 0,01 et 5% en poids, par rapport au poids total de la composition.

La composition tinctoriale selon l'invention peut contenir un ou plusieurs colorants directs additionnels différents du ou des colorants mixtes décrits auparavant.

On peut utiliser les colorants directs classiquement mis en oeuvre dans le domaine de la coloration des fibres kératiniques, et notamment des fibres kératiniques humaines.

A ce titre, on peut notamment citer les colorants nitrés de la série benzénique, les colorants additionnels directs azoïques, les colorants directs méthiniques. Ces colorants directs peuvent être de nature non ionique, anionique ou cationique. De préférence ces colorants directs additionnels sont de nature cationique.

Selon un mode de réalisation particulier de l'invention, la composition tinctoriale comprend une teneur en chacun des colorants directs variant de 0,001 à 10 % en poids par rapport au poids total de la composition tinctoriale.

La composition tinctoriale de l'invention peut aussi contenir une ou plusieurs bases d'oxydation et/ou un ou plusieurs coupleurs, conventionnellement utilisés pour la teinture de fibres kératiniques, et notamment des fibres kératiniques humaines, telles que notamment les cheveux.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

La ou les bases d'oxydation présentes dans la composition de l'invention sont en général utilisées chacune en quantité variant de 0,001 à 10 % en poids par rapport au poids total de la composition tinctoriale, de préférence variant de 0,005 à 6 % en poids.

Parmi les coupleurs utilisables, on peut notamment citer les métaphénylènediamines, les méta-aminophénols, les méta-diphénols, les coupleurs naphtaléniques, les coupleurs hétérocycliques et leur sels d'addition.

Dans la composition de la présente invention, le ou les coupleurs sont chacun généralement présents en quantité variant de 0,001 à 10 % en poids par rapport au poids total de la composition tinctoriale, de préférence variant de 0,005 à 6 % en poids.

D'une manière générale, les sels d'addition, notamment des bases d'oxydation et des coupleurs utilisables dans le cadre de l'invention, sont par exemple choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en $C_1$-$C_4$, comme par exemple, le monoéthyléther ou le monobutyléther du diéthylèneglycol, seuls ou en mélange.

Les solvants sont, de préférence, présents dans des proportions allant de 1 à 40 % en poids environ par rapport au poids total de la composition tinctoriale, et encore plus préférentiellement de 5 à 30 % en poids environ.

La composition tinctoriale conforme à l'invention peut également renfermer divers adjuvants utilisés classiquement dans les compositions pour la teinture des fibres kératiniques, notamment humains et en particulier les cheveux, tels que des agents tensioactifs anioniques, cationiques, non-ioniques, amphotères, zwitterioniques ou leurs mélanges ; des polymères anioniques, cationiques, non ioniques, amphotères, zwitterioniques ou leurs mélanges ; des agents épaississants minéraux ou organiques, et en particulier les épaississants associatifs polymériques anioniques, cationiques, non ioniques et amphotères ; des agents antioxydants ; des agents de pénétration ; des agents séquestrants ; des parfums ; des tampons ; des agents dispersants ; des agents de conditionnement tels que par exemple des silicones volatiles ou non volatiles, modifiées ou non modifiées ; des agents filmogènes ; des céramides ou pseudocéramides ; des agents conservateurs ; des agents opacifiants, etc.

Les adjuvants ci dessus sont en général présents en quantité variant, pour chacun d'eux, de 0,01 à 20 % en poids par rapport au poids de la composition.

La composition de l'invention peut en outre contenir au moins un agent oxydant.

Les agents oxydants classiquement utilisés pour la teinture d'oxydation des fibres kératiniques, notamment humaines, sont par exemple le peroxyde d'hydrogène, le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition de l'invention peut de plus comprendre au moins un agent alcalin qui peut être choisi parmi ceux utilisés classiquement dans le domaine cosmétique.

Parmi ces agents alcalins, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante :

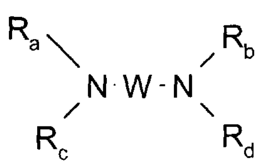
(A)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

Le pH de la composition tinctoriale de l'invention est de préférence compris entre 8 et 11.

Bien entendu, l'homme de l'art veillera à choisir ce ou ces éventuels composés complémentaires de manière

telle que les propriétés avantageuses attachées intrinsèquement à la composition selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, notamment humaines, et plus particulièrement des cheveux.

Le procédé de la présente invention est un procédé dans lequel on applique sur les fibres, sèches ou non, la composition selon la présente invention telle que définie précédemment.

Selon une première variante, la composition appliquée sur les fibres kératiniques, ne comprend pas d'agent oxydant. Cette variante est particulièrement appropriée lorsque la composition tinctoriale comprend au moins un colorant mixte selon l'invention et éventuellement au moins un colorant direct additionnel.

Selon une deuxième variante de l'invention, le procédé est mis en oeuvre avec au moins un agent oxydant. Cette deuxième variante est appropriée quelle que soit la nature des colorants présents (colorant mixte, colorant direct additionnel, bases d'oxydation et/ou coupleurs). Un tel procédé permet aussi d'obtenir un éclaircissement de la fibre traitée.

Selon cette deuxième variante, l'agent oxydant peut être ajouté à la composition tinctoriale au moment de l'emploi ou bien il peut encore être mis en oeuvre à partir d'une composition oxydante le comprenant, appliquée simultanément ou séquentiellement à la composition tinctoriale comprenant le colorant mixte. Dans ce dernier cas, l'agent oxydant est présent dans une composition différente de celle comprenant le colorant mixte.

Selon un mode de réalisation particulier, la composition comprenant le colorant mixte est mélangée, de préférence au moment de l'emploi, à une composition contenant, dans un milieu approprié pour la teinture, au moins un agent oxydant, cet agent oxydant étant présent en une quantité suffisante pour obtenir l'éclaircissement souhaité.

Le mélange obtenu est ensuite appliqué sur les fibres kératiniques.

Après un temps de pose suffisant pour obtenir la coloration désirée, habituellement variant de 3 à 50 minutes environ, de préférence 5 à 30 minutes environ, les fibres kératiniques sont de préférence rincées, puis lavées au shampooing, rincées à nouveau puis séchées ou laissées sécher.

Par ailleurs, de manière classique la composition est appliquée et laissée agir à une température allant de 15 à 80°C, de préférence de 15 à 40°C.

La composition oxydante peut également renfermer divers adjuvants utilisés classiquement dans les compositions pour la teinture des fibres kératiniques, notamment humaines, et tels que définis précédemment.

Le pH de la composition oxydante renfermant l'agent oxydant est tel qu'après mélange avec la composition tinctoriale, le pH de la composition résultante appliquée sur les fibres kératiniques (soit en d'autres termes la composition prête à l'emploi) varie de préférence de 7 à 12 environ, et encore plus préférentiellement de 8 à 11. Il peut être ajusté à la valeur désirée au moyen d'agent(s) acidifiant(s) ou alcalinisant(s).

Parmi les agents acidifiants, on peut citer, à titre d'exemples, les acides minéraux ou organiques comme l'acide chlorhydrique, l'acide orthophosphorique, l'acide sulfurique, l'acide acétique.

Pour ce qui concerne les composés alcalinisants, on pourra se reporter à la liste précédemment indiquée.

La composition prête à l'emploi, soit en d'autres termes la composition qui est finalement appliquée sur les fibres kératiniques, peut se présenter sous des formes diverses, telles que sous forme de liquides, de crèmes, de gels ou sous toute autre forme appropriée pour réaliser une teinture des fibres kératiniques, notamment des fibres kératiniques humaines, telles que les cheveux.

L'invention a aussi pour objet un dispositif à plusieurs compartiments dans lequel au moins un premier compartiment comprend une composition tinctoriale comprenant au moins un colorant mixte tel que décrit auparavant, et éventuellement au moins un colorant direct différent du colorant mixte, éventuellement au moins une base d'oxydation, éventuellement au moins un coupleur, et un autre compartiment comprend un agent oxydant.

Il est à noter que le colorant mixte, éventuellement le colorant direct additionnel, la ou les bases d'oxydation, le ou les coupleurs, peuvent se trouver dans un même compartiment ou dans plusieurs ; un même compartiment pouvant comprendre un seul type de colorant (mixte, direct additionnel, oxydation) ou une combinaison de plusieurs d'entre eux.

Ce dispositif peut être équipé d'un moyen permettant de délivrer sur les fibres à traiter, le mélange souhaité, tel que les dispositifs décrits dans le brevet FR 2 586 913.

Un dernier objet de la présente invention est constitué par des colorants mixtes en tant que tels, ainsi que leurs sels d'addition, tels qu'ils viennent d'être décrits, à l'exception de colorants mixtes à deux chromophores dont l'un est un composé nitré benzénique, l'autre un composé nitré benzénique ou anthraquinonique ; les deux chromophores étant reliés au moyen d'un atome d'azote par l'intermédiaire d'un bras de liaison constitué par un radical alkyle éventuellement interrompu par un atome d'azote porteur d'un ou de deux radicaux choisis parmi l'hydrogène, un radical alkyle en $C_1$-$C_4$, un radical hydroxyalkyle en $C_1$-$C_4$. De préférence, ne sont pas couverts par l'invention les colorants mixtes à deux chromophores dont l'un est un composé nitré benzénique, l'autre un composé nitré benzénique ou anthraquinonique ; les deux chromophores étant reliés par l'intermédiaire d'un bras de liaison constitué par un radical

alkyle éventuellement interrompu par un atome d'azote porteur d'un ou de deux radicaux choisis parmi l'hydrogène, un radical alkyle en $C_1$-$C_4$, un radical hydroxyalkyle en $C_1$-$C_4$. Ne sont de même pas compris dans l'invention les composés de formules suivantes :

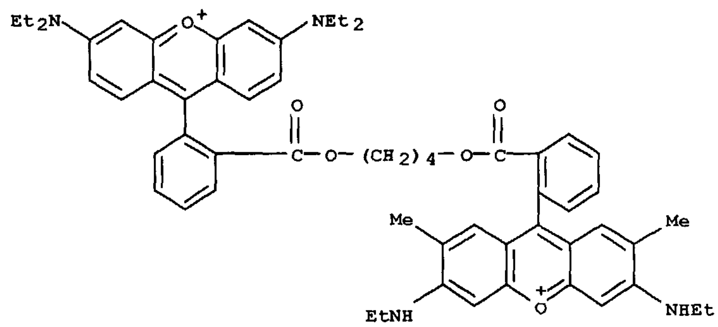

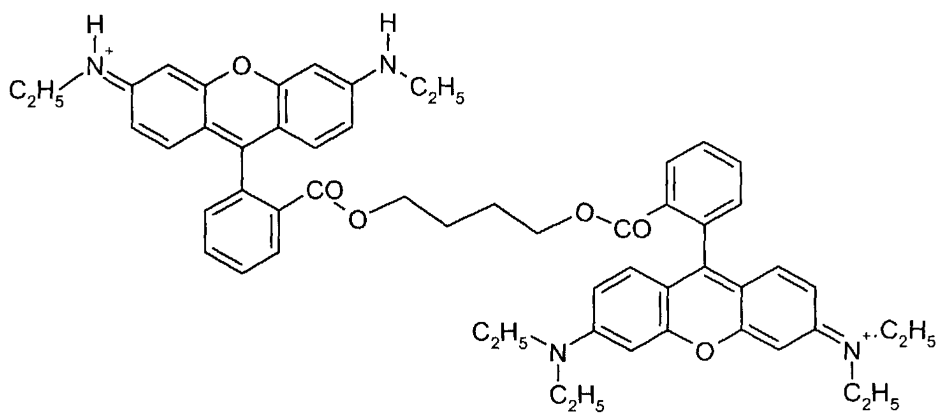

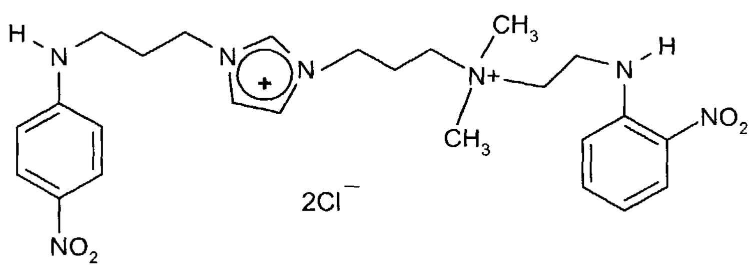

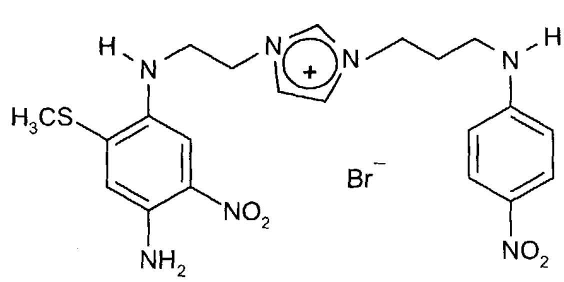

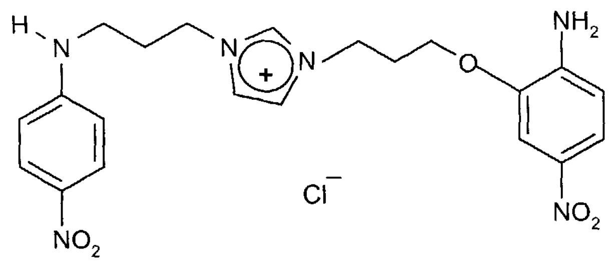

Il est de même précisé que la composition tinctoriale selon l'invention ne comprend de préférence pas les composés ci-dessus.

De préférence, le colorant mixte selon l'invention ne correspond pas à un dichromophore comprenant deux composés nitrés benzéniques, ou à un composé nitré benzénique et à un composé anthraquinonique.

A titre de colorant mixte particulier conforme à la présente invention, on peut citer les composés suivants, ainsi que ses sels d'addition :

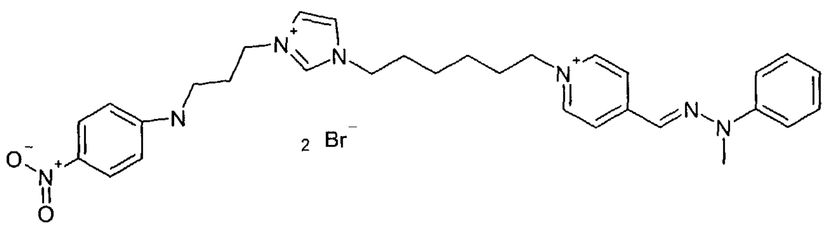

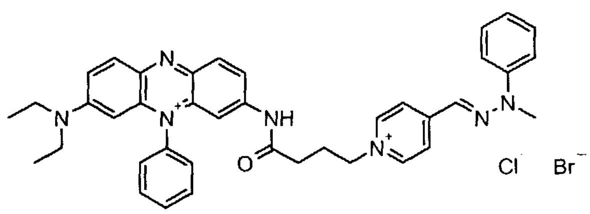

(III)

Des exemples concrets mais non limitatifs de la présente invention vont maintenant être présentés

## EXEMPLE 1

### Synthèse du colorant mixte

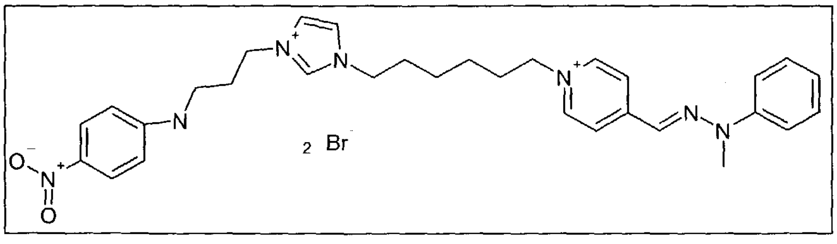

### Schéma réactionnel :

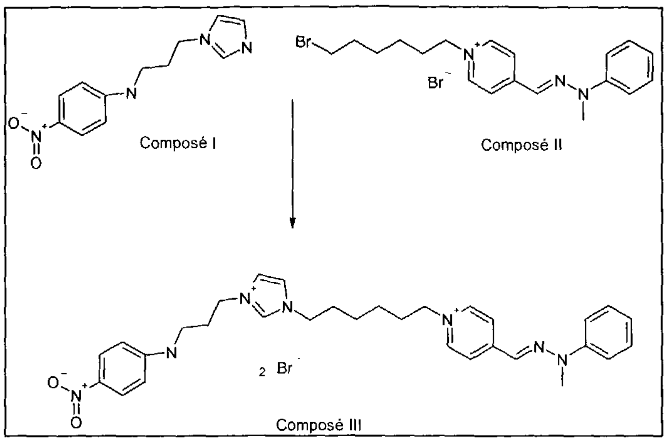


Les composés I (3,33g) et II (6,16g) sont mélangés dans le diméthylformamide (DMF) (100ml) et agités à 90°C pendant 18 heures.
Le mélange est versé sur de l'acétate d'éthyle (300ml) donnant une gomme.
La gomme est reprise dans le méthanol (100ml), filtré et évaporée à sec. On obtient des paillettes (6,0g).
Les spectres RMN et masse sont en accord avec la structure du produit attendu.

**Exemples de teinture**

**1- Composition de teinture**

Le colorant mixte obtenu à l'exemple précédent est formulé à $5,7.10^{-4}$ mol% dans la composition de coloration A.

| Composition A | |
|---|---|
| Alkyl ($C_8/C_{10}$ 50/50) polyglucoside en solution aqueuse à 60 % tamponnée | 10 g |
| Alcool benzylique | 10 g |
| Polyéthylène glycol 400 à 8 motifs d'oxyde d'éthylène | 12 g |
| Colorant de mixte l'exemple 1 | $5,7.10^{-4}$ mol |
| Ammoniaque à 20.5 % | 10 g |
| Eau déminéralisée | qsp 100 g |

Au moment de l'emploi, la composition A est mélangée avec de l'eau oxygénée 20 volumes (poids pour poids, pH = 3,5).
Le pH de la composition tinctoriale après mélange est compris entre 9,5 et 10.
Le mélange est ensuite appliqué sur des mèches de cheveux à 90% blancs naturels fortement décolorés (solubilité alcaline (SA) = 83%).
Le temps de pause sur mèches est de 20 min à température ambiante.
On obtient des mèches colorées.

**2/ Résistance aux shampoings**

On colore des mèches mèches de cheveux à 90% blancs naturels fortement décolorés (solubilité alcaline (SA) = 83%)d'une part avec une composition selon l'invention (voir le paragraphe précédent) et d'autre part avec une composition comparative comprenant un mélange équimolaire des colorants I et II constituant le colorant mixte :

Colorant jaune nitré (I) :

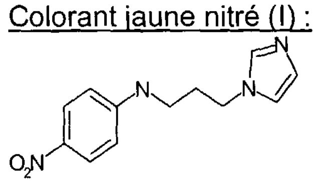

Colorant jaune hydrazone (II) :

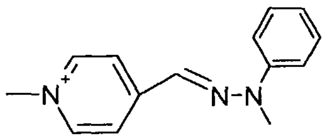

$CH_3SO_4^-$

Les mèches colorées subissent six shampooings, avec un séchage intermédiaire entre deux shampooings.

La couleur après les six shampooings est comparée à la couleur initiale de la mèche colorée, visuellement et par mesure colorimétrique (colorimètre CM2002 Minolta, illuminant D65-10° CSI).

La résistance aux shampooings est mesurée selon la formule de ΔE ci-dessous à partir des valeurs de L*a*b* mesurées avant ($L_0$*$a_0$*$b_0$*) et après les 6 shampooings ($L_1$*$a_1$*$b_1$*).

$$\Delta E = \sqrt{(L_1^*-L_0^*)^2+(a_1^*-a_0^*)^2+(b_1^*-b_0^*)^2}$$

Ainsi, plus ΔE est faible et meilleure est la résistance aux shampooings répétés.

Les résultats colorimétriques sont regroupés dans les tableaux 1 et 2.

Tableau 2

| **Composition selon l'invention** | | | | |
|---|---|---|---|---|
| **Type de cheveux** | **L*** | **a*** | **b*** | **Dégradation (ΔE)** |
| Colorant mixte avant shampooing | 63,81 | 7,04 | 73,39 | 3,23 |
| Colorant mixte après shampooing | 65,78 | 4,50 | 73,07 | |
| **Compositions comparatives :** | | | | |
| **Type de cheveux** | **L*** | **a*** | **b*** | **Dégradation (ΔE)** |
| Mélange des colorants I et II avant shampooing | 65,45 | 1,88 | 70,41 | 19,29 |
| Mélange des colorants I et II après shampooing | 68,85 | -2,47 | 51,93 | |

Ces tableaux indiquent que le colorant mixte est nettement plus résistant aux shampooings répétés que le mélange physique des deux colorants.

**EXEMPLE 2**

**Synthèse du colorant mixte :**

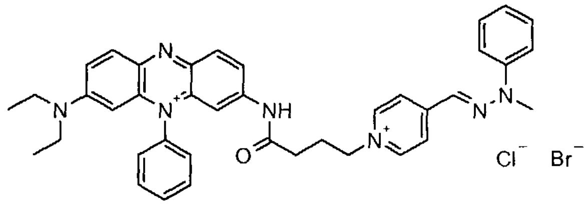

(III)

Schéma réactionnel :

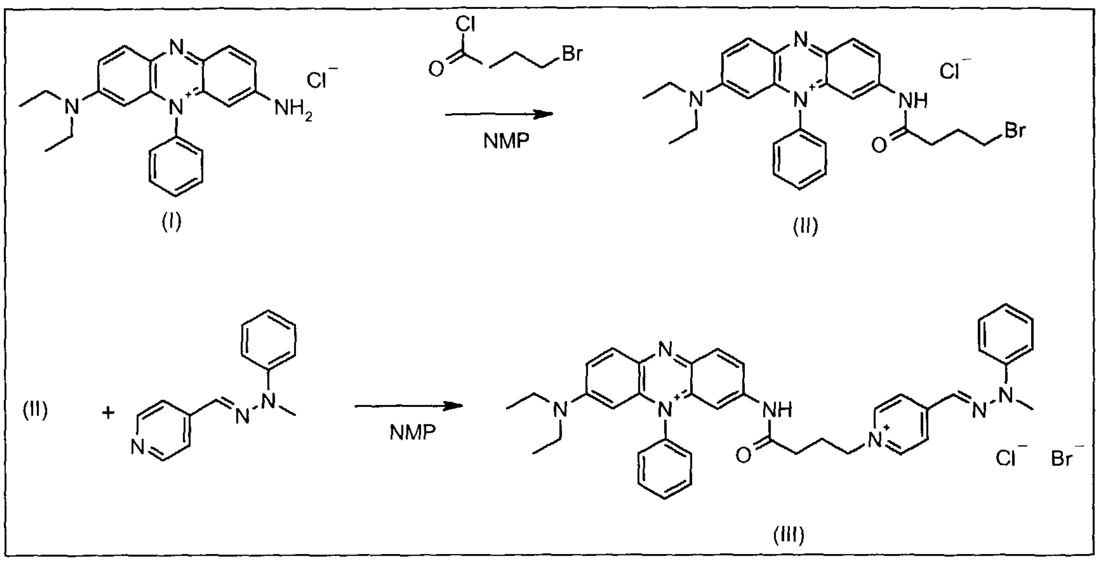


(I) (II)

(II) +

(III)

Mode opératoire :

Première étape de synthèse :

Dans un ballon de 100 mL, 2 g de Basic Violet 5 (composé (I)) pur, sont solubilisés dans 15 mL de N-méthylpyrrolidone (NMP), le mélange est maintenu sous agitation à température ambiante pendant une heure. 2 mL de chlorure de 4-bromobutyroyle sont introduits dans le mélange goutte à goutte, en trois minutes.

Le mélange réactionnel est maintenu agité à température ambiante pendant 16 heures, puis il est versé dans 200 mL d'acétone.

Le précipité violet-noir (composé (II)) ainsi obtenu est filtré, lavé par de l'acétone puis séchés sous vide.

On recueille 2.9 g de poudre violette.

Deuxième étape :

Dans un ballon de 100 mL, 2 g du composé (II) obtenu précédemment sont mélangés à 2 g d'isonicotinaldéhyde méthyl(phényl)hydrazone et 10 mL de NMP.

Le mélange est porté à 80°C pendant 24 heures.

Il est ensuite versé sur 200 mL d'acétone.

Un précipité violet se forme. Il est séché sous vide. Le produit obtenu est conforme au composé (III) (80% de pureté suivant en LCMS).

Le composé (III) est obtenu pur par lavages successifs d'une solution aqueuse de produit brut avec du 1-butanol puis concentration sous vide de cette solution.

**Exemple de teinture**

Une solution aqueuse de composé (III) (40 mg dans 100 mL d'eau) permet de teindre des mèches de cheveux gris dans une teinte marron très esthétique et résistante aux shampooings.

**Revendications**

1. Composition tinctoriale comprenant, dans un milieu approprié pour la teinture des fibres kératiniques, notamment humaines, au moins un colorant mixte comprenant au moins deux chromophores ; au moins l'un des chromophores étant choisi parmi les chromophores de la famille des composés azines cycliques et/ou nitrés (hétéro)aromatiques éventuellement associé à au moins un chromophore de la famille des méthines et/ou des carbonyles ; les chromophores étant liés entre eux par l'intermédiaire d'au moins un bras de liaison stoppant la délocalisation des électrons de chacun des chromophores ; à l'exception d'une composition comprenant un colorant mixte à deux chromophores dont l'un est un composé nitré benzénique, l'autre un composé nitré benzénique ou anthraquinonique, les deux chromophores étant reliés au moyen d'un atome d'azote par l'intermédiaire d'un bras de liaison constitué par un radical alkyle éventuellement interrompu par un atome d'azote porteur d'un ou de deux radicaux choisis parmi l'hydrogène, un radical alkyle en $C_1$-$C_4$, un radical hydroxyalkyle en $C_1$-$C_4$, et des composés de formules suivantes :

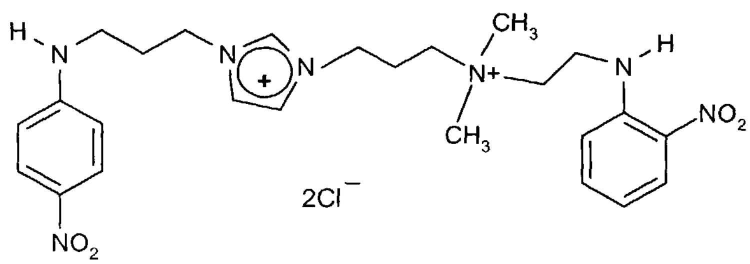

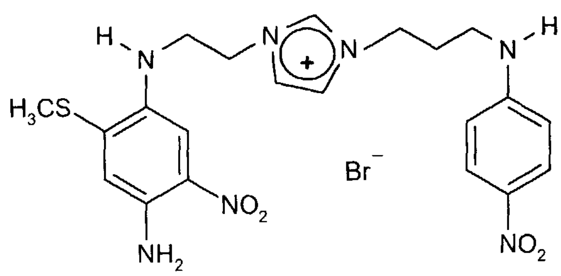

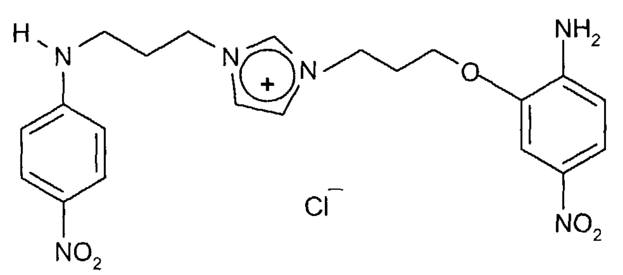

2. Composition selon la revendication précédente, **caractérisée en ce que** le colorant mixte comprend au moins deux chromophores différents dont l'un au moins porte une ou plusieurs charges cationiques.

3. Composition selon la revendication précédente, **caractérisée en ce que** les chromophores absorbent dans le domaine visible entre 400 et 800 nm.

4. Composition selon l'une quelconque des revendications précédentes, **caractérisée en ce que** le colorant mixte comprend deux à quatre chromophores, de préférence deux à trois chromophores.

5. Composition selon l'une quelconque des revendications précédentes, **caractérisée en ce que** le colorant mixte comprend deux chromophores.

6. Composition selon l'une des revendications précédentes, **caractérisée en ce que** le ou les chromophores cationiques sont des chromophores comprenant au moins un atome d'azote quaternisé.

7. Composition selon l'une quelconque des revendications précédentes, **caractérisée en ce que** le colorant mixte comprend au moins un chromophore de la famille des azines cycliques.

8. Composition selon la revendication précédente, **caractérisée en ce que** le chromophore de la famille des azines cycliques est choisi parmi les azine, xanthène, thioxanthène, fluorindine, acridine, (di)oxazine, (di)thiazine, pyronine.

9. Composition selon la revendication précédente, **caractérisée en ce que** le chromophore de la famille des azines est un radical issu de colorants formule suivante (I) ainsi que ses formes tautomères :

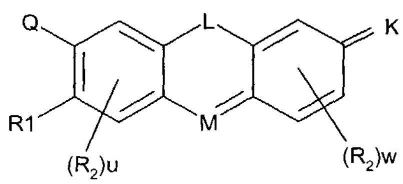

formule dans laquelle :

L, représente un hétéroatome de préférence choisi parmi l'oxygène, le soufre ; un groupe NH ; un groupe N-$R_3$,
M représente un hétéroatome de préférence choisi parmi l'oxygène, le soufre, l'azote ; un groupe $N^+$-$R_3$ ; un groupe CH ; un groupe C-$R_4$ ;
Q, K, identiques ou non, représentent un groupement hydroxyle ; un radical amino ; un radical amino substitué par un ou plusieurs radicaux alkyle, identiques ou non, linéaires ou ramifiés, en $C_1$-$C_8$, éventuellement porteurs d'au moins un groupement hydroxyle, un radical aryle éventuellement substitué, un radical alkyl($C_1$-$C_8$)aryle éventuellement substitué ; un groupement ammonium de type N+$(R_5)_t$ avec t égal à 2 pour K et à 3 pour Q ;
$R_5$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl($C_1$-$C_8$)aryle la partie aryle étant éventuellement substituée ; un radical alkyle en $C_1$-$C_8$, linéaire ou ramifiée, éventuellement substituée, un radical alcoxy, linéaire ou ramifié, en $C_1$-$C_8$, éventuellement substitué ;
à la condition que Q et K ne représentent pas à la fois un groupement ammonium de type $N^+(R_5)_t$ ;
$R_1$, $R_2$, identiques ou non, représentent un atome d'hydrogène ; un radical alkyle, linéaire ou ramifié, en $C_1$-$C_8$ éventuellement substitué ; un radical amino ; un radical amino substitué ou non par un ou plusieurs radicaux, identiques ou non, choisis parmi les radicaux alkyle, linéaires ou ramifiés, en $C_1$-$C_8$ éventuellement substitués, les radicaux phényle éventuellement substitués ; un atome d'halogène de préférence le chlore, le fluor ;
au cas où Q représente un radical amino substitué ou non, ou un groupement hydroxyle, $R_1$ peut représenter un radical alkylamino ou alcoxy formant avec l'atome d'azote ou d'oxygène du radical J, un cycle à 6 chaînons, éventuellement condensé avec un radical aromatique, ledit radical aromatique étant éventuellement substitué par au moins un groupement amino ou amino substitué ou non par un ou plusieurs radicaux identiques ou non, choisis parmi les radicaux alkyle, linéaires ou ramifiés, en $C_1$-$C_8$ éventuellement substitués, les radicaux phényle éventuellement substitués ;
$R_3$, $R_4$, identiques ou non, représentent un radical alkyle, linéaire ou ramifié en $C_1$-$C_8$ éventuellement substitué, de préférence par au moins un hydroxyle ; au moins un radical alcoxy, linéaire ou ramifié en $C_1$-$C_8$ ; au moins un radical amino ; au moins un radical amino substitué par un ou plusieurs radicaux alkyles, identiques ou

non, linéaires ou ramifiés, en $C_1$-$C_8$, éventuellement porteurs d'au moins un radical hydroxyle ;
$R_3$, $R_4$, identiques ou non, peuvent aussi représenter un radical aryle, de préférence en $C_6$, éventuellement substitué, de préférence par au moins un radical alkyle, linéaire ou ramifié, en $C_1$-$C_8$ ; par au moins un groupement hydroxyle ; par au moins un radical alcoxy, linéaire ou ramifié, en $C_1$-$C_8$ ; par au moins un radical amino ; par au moins un radical amino substitué par un ou plusieurs radicaux alkyle, identiques ou non, linéaires ou ramifiés, en $C_1$-$C_8$, éventuellement porteurs d'au moins un groupement hydroxyle ; un atome d'halogène et de préférence le chlore ou le fluor ; par au moins un groupement nitro ; par au moins un groupement cyano, de préférence $R_3$ et $R_4$, identiques ou non, représentent un radical alkyle, linéaire en $C_1$-$C_4$, un radical aryle éventuellement substitué ;
le coefficient u est égal à 2 ; w est égal à 3.

**10.** Composition selon l'une quelconque des revendications précédentes, **caractérisée en ce que** le chromophore de la famille des nitrés (hétéro)aromatiques est un radical issu de colorants correspondant aux formules (II) et (III) ci-dessous, ainsi que leurs formes tautomères :

| | |
|---|---|
| benzénique | 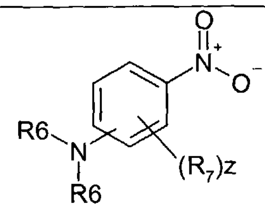 (II) |
| Pyridinique | 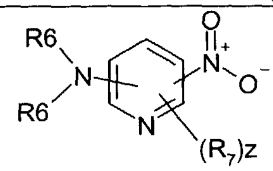 (III) |

Formules dans lesquelles

$R_6$, identiques ou non, représentent un atome d'hydrogène ; un radical alkyle, linéaire ou ramifié, en $C_1$-$C_8$, éventuellement porteur d'au moins un groupement hydroxyle ; un radical aryle éventuellement substitué, un radical alkyl($C_1$-$C_8$)aryle la partie aryle étant éventuellement substituée ; un radical alkyle en $C_1$-$C_8$, linéaire ou ramifiée, éventuellement substituée, un radical alcoxy, linéaire ou ramifié, en $C_1$-$C_8$, éventuellement substitué ;
$R_7$, identiques ou différents, représentent un atome d'hydrogène ; alkyle linéaire ou ramifié en $C_1$-$C_4$, éventuellement substitué ; un radical alcoxy linéaire ou ramifié en $C_1$-$C_8$, éventuellement substitué ; un radical aryle en $C_6$ éventuellement substitué ; un radical amino ; un radical amino substitué par un ou plusieurs radicaux choisis parmi les radicaux alkyle, identiques ou non, linéaires ou ramifiés, en $C_1$-$C_8$, de préférence en $C_1$-$C_4$, éventuellement substitués, de préférence par au moins un groupe hydroxyle, alcoxy, linéaire ou ramifié en $C_1$-$C_4$, thioalkyle linéaires ou ramifiés en $C_1$-$C_4$, alkylsulfonamido linéaires ou ramifiés en $C_1$-$C_4$, parmi les radicaux aryle en $C_6$ éventuellement substitué et parmi les radicaux hétéroaryle comprenant 5 à 6 chaînons éventuellement substitués ; un groupe hydroxyle ; un groupe nitro ; un groupe cyano.

**11.** Composition selon l'une quelconque des revendications précédentes, **caractérisée en ce que** les chromophores de type méthines, sont choisis parmi les composés comprenant au moins un enchaînement choisi parmi >C=C< et -N=C< dont les deux atomes ne sont pas simultanément engagés dans un cycle ; sachant qu'il n'est pas exclu que l'un des atomes d'azote ou de carbone des enchaînements peut être engagé dans un cycle.

**12.** Composition selon l'une des revendications précédentes, **caractérisée en ce que** les chromophores de type méthine sont des radicaux issus de chromophores de la famille des méthine, azométhine, mono- et di- arylméthane, indamines, indophénols, indoanilines, carbocyanines, azacabocyanines et leurs isomères, diazacarbocyanines et leurs isomères, tétraazacarocyanines, hémicyanines, ainsi que, le cas échéant, leurs isomères.

**13.** Composition selon l'une des revendications précédentes, **caractérisée en ce que** les chromophores de type méthine sont des radicaux issus de chromophores de formule suivante (IV) ainsi que ses formes tautomères :

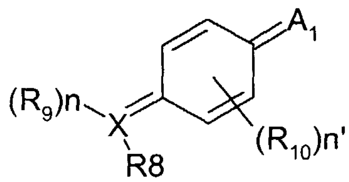

Formule (IX) dans laquelle :

- $R_8$, $R_9$, identiques ou non, représentent un atome d'hydrogène ; un radical aryle en $C_6$-$C_{30}$ un radical alkyl ($C_1$-$C_8$)aryle, la partie aryle étant éventuellement substituée par un ou plusieurs groupements, identiques ou non, choisis de préférence parmi les groupements hydroxyle, alcoxy, linéaire ou ramifié, substitué ou non, en $C_1$-$C_4$, amino, amino substitué par un ou plusieurs radicaux alkyles, identiques ou non, linéaires ou ramifiés, en $C_1$-$C_4$ éventuellement porteurs d'au moins un groupement hydroxyle, les atomes d'halogène, de préférence le chlore ; un radical hétérocyclique, choisi notamment parmi les hétérocycles thiophène, furane, pipéronyle, indole, indoline, pyridine, carbazole, déhydroquinoléïne, chromone ;
- $R_8$, $R_9$, ne peuvent en outre représenter simultanément ni un radical aromatique, ni un radical hétéroaromatique ;
- $R_{10}$, identiques ou différents, représentent un atome d'hydrogène ; un radical alkyle, linéaire ou ramifié, en $C_1$-$C_8$ éventuellement substitué ; un radical aryle en $C_6$-$C_{30}$, éventuellement substitué ; un radical amino ; un radical amino substitué par ou plusieurs radicaux alkyle, linéaires ou ramifiés, en $C_1$-$C_4$, éventuellement porteurs d'au moins un groupement hydroxyle ; un groupement hydroxyle ; un radical alcoxy, linéaire ou ramifié, en $C_1$-$C_8$, éventuellement porteur d'au moins un groupement hydroxyle, alcoxy en $C_1$-$C_4$ ; un atome d'halogène et de préférence le chlore ;
- X représente un atome d'azote, un atome de carbone ;
- le coefficient n représente 0 lorsque X est un atome d'azote, et 1 lorsque X est un atome de carbone ;
- le coefficient n' est égal à 4 ;
- $A_1$ représente un groupement amino ; un groupement amino substitué par un ou plusieurs radicaux alkyles en $C_1$-$C_8$, identiques ou non, linéaires ou ramifiés, éventuellement porteurs d'au moins un groupement hydroxyle, un groupement ammonium $N^+(R_{11})_2$ ; $R_{11}$, identiques ou non, représentent un radical alkyle en $C_1$-$C_8$ éventuellement substitué ; un radical aryle en $C_6$ éventuellement substitué, de préférence par un ou plusieurs hydroxyle, atomes d'halogène, de préférence le chlore, le fluor, groupes nitro, groupes cyano, alcoxy, linéaires ou ramifiés, en $C_1$-$C_4$, monohydroxy alcoxy en $C_1$-$C_4$, linéaires ou ramifiés, polyhydroxyalcoxy, en $C_2$-$C_4$, linéaires ou ramifiés, amino substitués ou non par un ou plusieurs radicaux alkyle, hydroxyalkyle, identiques ou différents, en $C_1$-$C_4$, linéaires ou ramifiés ;

Conviennent aussi ceux issus de la formule suivante (V) :

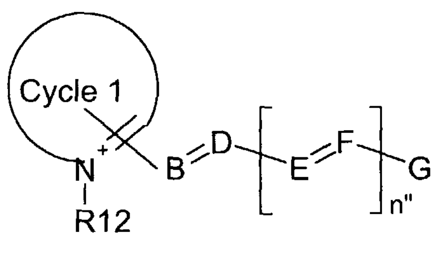

et le cas échéant leurs formes tautomères ; formule dans laquelle

B, D, E, et F, identiques ou différents, représentant un atome d'azote ou un groupe C-$R_{13}$,
$R_{13}$, identiques ou non, représentent un atome d'hydrogène, un radical alkyle en $C_1$-$C_8$ éventuellement substitué, de préférence par au moins un hydroxyle ; un radical alcoxy, linéaire ou ramifié, en $C_1$-$C_4$ ; un radical amino ; un radical amino substitué par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué, un radical hétéroaryle comprenant de 5 à 12 chaînons, éventuellement substitué ;
n" = 0 ou 1 ;
G représentant un Cycle 4, ou les restes :

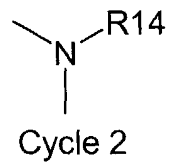

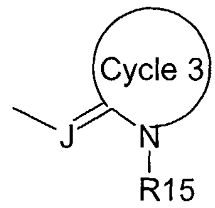

formules dans lesquelles :

$R_{12}$ et $R_{15}$ représentent indépendamment l'un de l'autre, un radical alkyle, linéaire ou ramifié, en $C_1$-$C_8$ éventuellement substitué ; un radical benzyle éventuellement substitué ;
$R_{14}$ représente un atome d'hydrogène, un radical alkyle en $C_1$-$C_8$ éventuellement substitué, de préférence par au moins un hydroxyle, un radical alcoxy, linéaire ou ramifié, en $C_1$-$C_4$, un radical amino, un radical amino substitué par un ou plusieurs radicaux alkyle, identiques ou non, linéaires ou ramifiés, en $C_1$-$C_4$, éventuellement porteurs d'au moins un groupe hydroxyle ; un radical aryle en $C_6$ éventuellement substitué ; un radical hétéroaryle en $C_2$-$C_{12}$ éventuellement substitué ;
**J** représente un atome d'azote ou un groupe C-$R_{16}$ ; avec $R_{16}$ ayant la même signification que $R_{13}$ ;
**Cycle 1** étant choisi parmi les radicaux hétéroaromatiques à 5 à 12 chaînons, portant au moins une charge cationique sur un atome d'azote et comprenant éventuellement au moins un autre hétéroatome choisi parmi l'azote, l'oxygène et/ou le soufre ; lesdits radicaux étant éventuellement substitués par au moins un radical alkyle, linéaire ou ramifié, en $C_1$-$C_8$ substitué ou non ; au moins un radical alcoxy, linéaire ou ramifié, en $C_1$-$C_8$ substitué ou non ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; au moins un radical alcoxycarbonyle, linéaire ou ramifié, en $C_1$-$C_8$ ; au moins un nitro ; au moins un cyano ; au moins un radical alkylsulfonamido linéaire ou ramifié, en $C_1$-$C_{12}$ (alkyl-$SO_2$-NH-) ; alkylsulfamoyle linéaire ou ramifié, en $C_1$-$C_{12}$ (alkyl-NH-$SO_2$-) ;
**Cycle 2** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons ; au moins un groupement hydroxyle ;
**Cycle 3** étant choisi parmi les radicaux hétéroaromatiques à 5 ou 6 chaînons comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical aromatique en $C_5$-$C_6$ ; au moins un groupement hydroxyle ; au moins un radical alcoxycarbonyle linéaire ou ramifié en $C_1$-$C_8$ ; nitro ; cyano ; au moins un radical alkylsulfonamido linéaire ou ramifié en $C_1$-$C_{12}$ (alkyl-$SO_2$-NH-) ; au moins un radical alkylsulfamoyle linéaire ou ramifié en $C_1$-$C_{12}$ (alkyl-NH-$SO_2$-) ;
**Cycle 4** étant choisi parmi les radicaux aromatiques en $C_6$-$C_{12}$ ; les radicaux hétéroaromatiques à 5 à 12 chaînons, comprenant au moins un hétéroatome choisi parmi l'azote, l'oxygène et/ou le soufre ; lesdits radicaux étant éventuellement substitués par au moins un radical alkyle, linéaire ou ramifié, en $C_1$-$C_8$ ; au moins un radical alcoxy, linéaire ou ramifié, en $C_1$-$C_8$ ; au moins un radical amino ; au moins un radical amino substitué par un ou plusieurs groupements alkyle, identiques, ou non, linéaires ou ramifiés, en $C_1$-$C_8$, éventuellement porteurs d'au moins un groupement hydroxyle ; au moins un radical (hétéro)aromatique comprenant de préférence 5 à 6 chaînons; au moins un groupement hydroxyle ;

à la condition que lorsque n" vaut 1 et G représente un cycle, alors B, D, E et F ne peuvent représenter simultanément un atome d'azote ; et que lorsque n" vaut 0 et G représente un cycle, alors B et D ne représentent pas simultanément un atome d'azote.

**14.** Composition selon l'une quelconque des revendications précédentes, **caractérisée en ce que** le colorant mixte

comprend au moins un chromophore de la famille des carbonyles.

**15.** Composition selon la revendication précédente, **caractérisée en ce que** le chromophore de la famille des carbonyles est un radical issu de colorants de la famille les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

**16.** Composition selon l'une quelconque des revendications 14 ou 15, **caractérisée en ce que** le chromophore de la famille des carbonyles est un radical issu de colorants de la formule suivante (VI) :

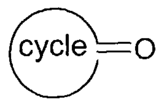

Formule dans laquelle le cycle représente un cycle à 5 ou 6 chaînons, dont éventuellement au moins l'un d'entre eux est remplacé par un hétéroatome choisi parmi l'oxygène, l'azote, le soufre, ou par une fonction carbonyle additionnelle ; ledit cycle étant éventuellement substitué par un radical ou plusieurs radicaux choisis parmi les radicaux alkyle, linéaire ou ramifié, en $C_1$-$C_8$, éventuellement substitués ; par un ou plusieurs radicaux hydroxyle ; les atomes d'halogène comme de préférence le chlore ; les groupements nitro, cyano, amino, alkylamino ; ledit cycle étant éventuellement condensé avec un ou plusieurs cycles aromatiques en $C_6$, ce ou ces cycles pouvant eux-mêmes être condensés à un ou plusieurs cycles aromatiques dont éventuellement au moins l'un des atomes de carbone est remplacé par un ou plusieurs hétéroatomes choisis parmi l'oxygène, l'azote ou le soufre.

**17.** Composition selon l'une quelconque des revendications précédentes, **caractérisée en ce que** le bras de liaison est cationique ou non cationique.

**18.** Composition selon l'une quelconque des revendications précédentes, **caractérisée en ce que** le bras de liaison est choisi parmi une chaîne hydrocarbonée en $C_1$-$C_{20}$, de préférence en $C_1$-$C_{12}$, linéaire ou ramifiée, de préférence une chaîne alkyle, un ou plusieurs des atomes de carbone de ladite chaîne pouvant chacun être remplacés par un hétéroatome tel que le soufre, l'azote, l'oxygène, par un hétérocycle comprenant 5 ou 6 chaînons, saturé ou non, comprenant de préférence au moins deux atomes d'azote ; ladite chaîne hydrocarbonée pouvant être insaturée ou contenir un radical arylène ; parmi un radical arylène ; parmi un radical divalent téréphtalamide ; parmi un radical divalent ou trivalent, par exemple de type triazine.

**19.** Composition selon l'une quelconque des revendications précédentes, **caractérisée en ce que** la teneur en colorant mixte varie de 0,001 à 20% en poids, plus particulièrement, de 0,005 à 10% en poids, et de préférence, de 0,01 et 5% en poids, par rapport au poids total de la composition.

**20.** Composition selon l'une quelconque des revendications précédentes, **caractérisée en ce qu'**elle comprend un ou plusieurs colorants directs additionnels autres que ledit colorant mixte.

**21.** Composition selon la revendication précédente, **caractérisée en ce que** la teneur en chacun des colorants directs additionnels est varie de 0,001 à 10 % en poids par rapport au poids total de la composition tinctoriale.

**22.** Composition selon l'une quelconque des revendications précédentes, **caractérisée en ce qu'**elle comprend au moins une base d'oxydation choisie parmi les paraphénylènediamines, les bis-phénylalkylènediamines, les para-aminophénols, les ortho-aminophénols, les bases hétérocycliques et leurs sels d'addition.

**23.** Composition selon l'une quelconque des revendications précédentes, **caractérisée en ce qu'**elle comprend au moins un coupleur choisi parmi les métaphénylènediamines, les méta-aminophénols, les métadiphénols, les coupleurs naphtaléniques, les coupleurs hétérocycliques et leurs sels d'addition.

**24.** Composition selon l'une des revendications 22 ou 23, **caractérisée en ce que** la teneur de chacune des bases d'oxydation est varie de 0,001 à 10 % en poids par rapport au poids total de la composition tinctoriale et la teneur de chacun des coupleurs varie de 0,001 à 10 % en poids par rapport au poids total de la composition tinctoriale.

**25.** Composition selon l'une quelconque des revendications précédentes, **caractérisée en ce que** la composition comprend au moins un agent oxydant.

**26.** Composition selon la revendication précédente, **caractérisée en ce que** l'agent oxydant est choisi parmi le peroxyde d'hydrogène, les peroxydes de métaux alcalins ou alcalino-terreux, le peroxyde d'urée, les bromates ou ferricyanures de métaux alcalins, les persels, les enzymes telles que les peroxydases et les oxydo-réductases à deux ou à quatre électrons, et de préférence le peroxyde d'hydrogène.

**27.** Composition selon l'une quelconque des revendications précédentes, **caractérisée en ce que** le pH est compris entre 8 et 11.

**28.** Procédé de coloration de fibres kératiniques, notamment humaines, consistant à mettre en oeuvre les étapes suivantes :

a) on applique la composition selon l'une quelconque des revendications 1 à 24, sur de fibres sèches ou non, éventuellement en présence d'au moins un agent oxydant,
b) on laisse poser pendant une durée suffisante pour obtenir la coloration souhaitée,
c) on rince éventuellement les fibres,
d) on lave les fibres et on les rince,
e) on sèche ou on laisse sécher les fibres.

**29.** Dispositif à plusieurs compartiments dans lequel au moins un premier compartiment contient une composition tinctoriale comprenant au moins un colorant mixte tel que décrit dans l'une des revendications 1 à 24, et éventuellement au moins un colorant direct additionnel différent du colorant mixte, éventuellement au moins une base d'oxydation, éventuellement au moins un coupleur, et un autre compartiment contenant un agent oxydant.

**30.** Colorant mixte tel que décrit dans l'une quelconque des revendications 1 à 18, et ses sels d'addition, à l'exception de colorants mixtes à deux chromophores dont l'un est un composé nitré benzénique, l'autre un composé nitré benzénique ou anthraquinonique ; les deux chromophores étant reliés au moyen d'un atome d'azote par l'intermédiaire d'un bras de liaison constitué par un radical alkyle éventuellement interrompu par un atome d'azote porteur d'un ou de deux radicaux choisis parmi l'hydrogène, un radical alkyle en $C_1$-$C_4$, un radical hydroxyalkyle en $C_1$-$C_4$ et à l'exception des composés de formules suivantes :

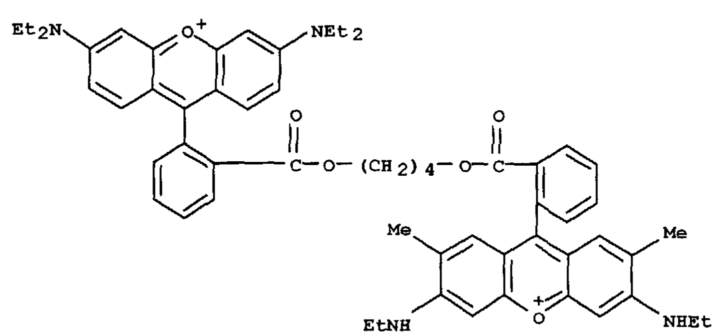

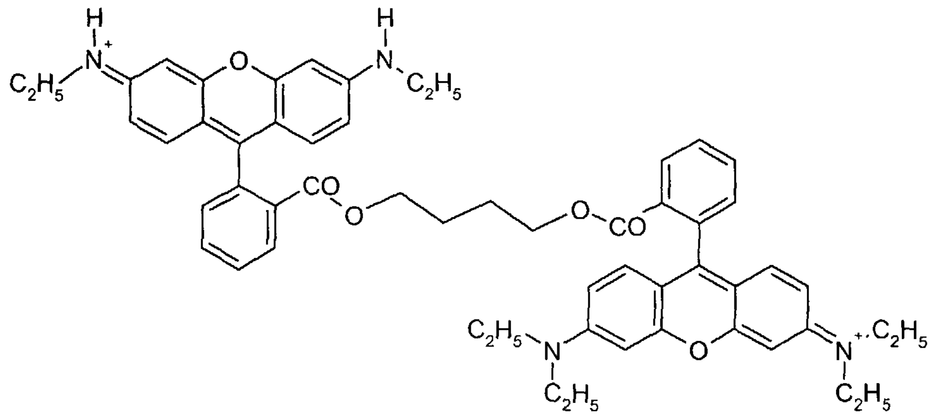

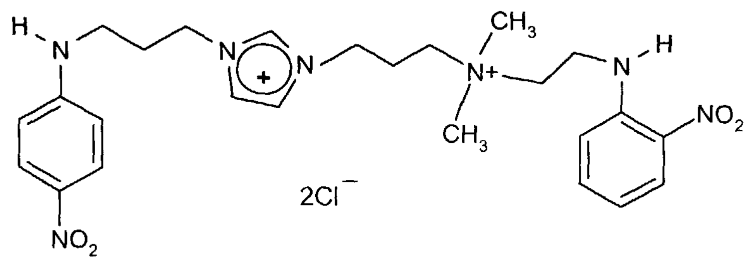

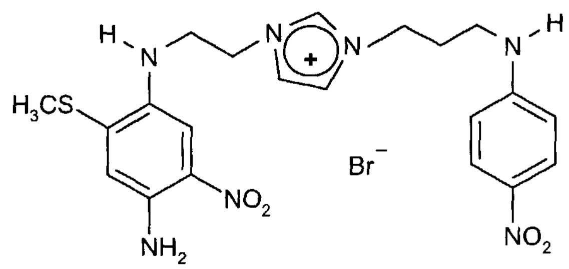

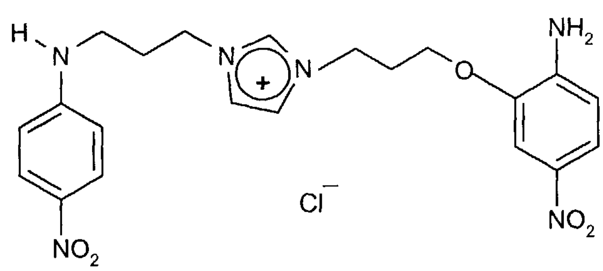

**31.** Colorant mixte selon la revendication précédente, **caractérisé en ce qu'**il correspond aux formules suivantes, ainsi que ses sels d'addition :

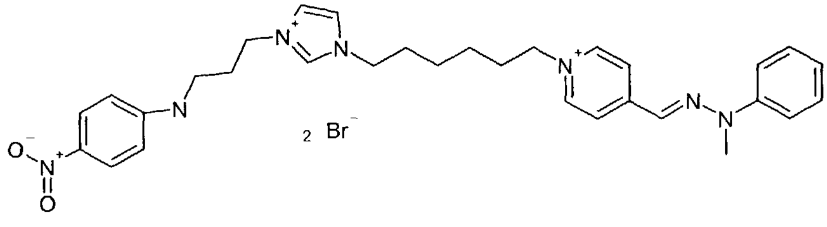

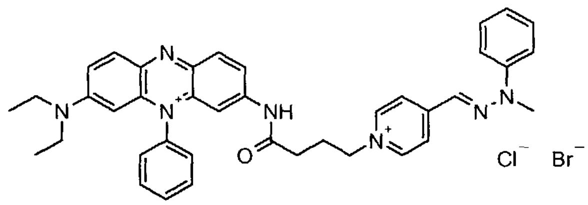

(III)